# EUROPÄISCHE PATENTSCHRIFT

(11) **EP 2 222 327 B1**
(45) Veröffentlichungstag und Bekanntmachung des Hinweises auf die Patenterteilung: **15.02.2017**
(21) Anmeldenummer: 08866728.2
(22) Anmeldetag: 20.12.2008
(51) Int. Cl.: A61K 47/32, A61K 38/18, A61K 9/00

(54) **TOPISCHE ANWENDUNG UND FORMULIERUNG VON ERYTHROPOIETIN FÜR DIE WUNDHEILUNG DER HAUT**
TOPICAL APPLICATION AND FORMULATION OF ERYTHROPOIETIN FOR SKIN WOUND HEALING
APPLICATION TOPIQUE ET FORMULATION D'ÉRYTHROPOÏÉTINE POUR LA CICATRISATION DE LA PEAU

(30) Priorität: 28.12.2007 EP 07025167
(43) Veröffentlichungstag der Anmeldung: 01.09.2010
(73) Patentinhaber: Bader, Augustinus, 04668 Parthenstein / OT Klinga (DE)
(72) Erfinder: Bader, Augustinus, 04668 Parthenstein / OT Klinga (DE)
(74) Vertreter: Benz, Jürgen
(86) Internationale Anmeldenummer: PCT/EP2008/010978
(87) Internationale Veröffentlichungsnummer: WO 2009/083203

(56) Entgegenhaltungen:
- WO-A-93/25221
- WO-A-97/07788
- WO-A-03/053471
- WO-A-2004/001023
- WO-A-2005/063965
- WO-A-2005/070451
- WO-A-2006/089056
- SHIBASHISH GIRI ET AL: 'Skin regeneration in deep second-degree scald injuries either by infusion pumping or topical application of recombinant human erythropoietin gel' DRUG DESIGN, DEVELOPMENT AND THERAPY 01 Mai 2015, Seiten 2565 - 2579, XP055283765 DOI: 10.2147/DDDT.S79425
- F. F. Wang ET AL: "Some Chemical Properties of Human Erythropoietin", Endocrinology, vol. 116, no. 6, 1 June 1985 (1985-06-01), pages 2286-2292, XP055283766,

## Beschreibung

### TECHNISCHES GEBIET DER ERFINDUNG:

Die Erfindung betrifft die Verwendung von Erythropoietin (EPO), insbesondere EPO in einer den Wirkstoff stabilisierenden pharmazeutischen Zubereitung zur topischen Behandlung von traumatisierter Haut, insbesondere zur Wundheilung bei mechanischen oder pathologischen Verletzungen oder bei Verbrennungen. Die Erfindung ist insbesondere gerichtet auf die Verwendung von EPO in einer erfindungsgemäßen stabilisierten Form auf Basis von hydrophilen Polymeren zur Stimulierung der Differenzierung und des Wachstums von bestimmten Zelltypen, beispielsweise gewebespezifische Vorläuferzellen, die keine vaskulären Vorläuferzellen sind, oder von adulten gewebespezifischen Zellen, die keine Endothelzellen sind, die jeweils im unmittelbaren Wundbereich bei derartigen Hautverletzungen anzutreffen sind.

Die Erfindung betrifft insbesondere auch spezielle viskose oder gelartige Formulierungen auf Basis von Polysacchariden, insbesondere Cellulose-Derivaten, welche EPO enthalten und dieses zu stabilisieren und langsam und gleichmäßig an die Wunde abzugeben vermögen.

### TECHNISCHER HINTERGRUND DER ERFINDUNG

Erythropoetin (EPO) ist ein Glykoprotein-Hormon, das die Bildung der Erythrozyten aus Vorgängerzellen im Knochenmark (Erythropoese) steuert. EPO bindet dabei an seinen Rezeptor (EPO-R), der auf allen hämatopoietischen Zellen exprimiert wird.

Bei Erwachsenen wird Erythropoietin hauptsächlich in der Niere gebildet, und zwar in den Endothelzellen der peritubulären Kapillaren. Geringere Mengen werden auch in den Leberzellen (Hepatozyten) synthetisiert.

Die Hauptwirkung von EPO besteht also darin, die Anzahl der roten Blutkörperchen im Blut zu erhöhen, was zu einer erhöhten Sauerstoffaufnahme führt.

In den letzten Jahren, wurde von diversen Autoren berichtet, dass EPO auch eine nicht-hämatopoietische Wirkung ausübt, und das EPO-R entsprechend auch von bestimmten nicht-hämatopoietischen Zellen exprimiert wird. So wird von einer Stimulierung durch EPO von Nervenzellen, neuronale Zellen des Gehirn und Endothelzellen berichtet, wobei dies in einigen Fällen mit einer direkten Expression des hämatopoietischen EPO Rezeptors verbunden ist. In anderen Fällen wird das Vorhandensein eines weiteren, nicht-hämatopoietischen Rezeptors prognostiziert, der aber noch nicht nachgewiesen wurde.

Insbesondere der noch nicht sehr lange bekannten nicht-hämatopoietischen Wirkung von Erythropoietin (EPO) im Zusammenhang beispielsweise mit der angeregten Bildung und Regeneration von Endothel- und Gewebezellen, wie Bindegewebe, Muskelgewebe, Epithelgewebe und Nervengewebe, wird zunehmend Bedeutung beigemessen.

So beschreibt die WO 2004/001023 unter anderen die Verwendung von EPO und TPO zur Anregung der Gefäßneubildung und Geweberegeneration und Verbesserung der Wundheilung, z.B. nach Operationen oder Verletzungen.

In der WO 2005/063965 wird die Verwendung von EPO zur gezielten strukturell gelenkten Regeneration von traumatisiertem Gewebe gelehrt, bei welcher nicht nur ein Endothelzellwachstum stimuliert, sondern auch die Parenchymregeneration und die Ausbildung der Wandstrukturen gefördert wird, so dass ein koordiniertes dreidimensionalen Wachstum zum Aufbau eines funktionsfähigen Gewebes, Organs oder Teilen davon von statten geht.

Haroon et el. (American J. Pathol. 2003, 163, 993) diskutieren die neue Rolle von EPO im Rahmen der durch Fibrin induzierten Wundheilungsprozesse.

In einem Review-Artikel diskutieren Brines und Cerami (Kidney International, 2006) die Rolle von EPO bei der Protektion von Gewebe.

Erythropoietin, und EPO-Derivate oder auch EPO-Mimetika scheinen bei systemischer Anwendung also hervorragend geeignet zu sein, um bei Verletzungen der Haut, der Schleimhaut, bei offenen Haut- und Fleischwunden oder auch bei Hautirritationen durch Verbrennungen oder Verbrühungen die Neubildung und Regeneration des betroffenen Gewebes gezielt zu initiieren und zu steuern und letztlich die Heilung fördern und beschleunigen zu können.

Die WO 2005/070450 sowie weitere Arbeiten der betreffenden Erfinder beschreibt die Verwendung von EPO bei der Regeneration von Gefäßen und Gewebe mit eine wöchentlichen Dosis von unter 90 IE/kg Körpergewicht (= KGW) unter anderem auch für den Bereich der Wundversorgung. Auch wenn hierbei theoretisch von der möglichen topischen Anwendung gesprochen wird, so wird dennoch herausgestellt, dass die systemische Applikation bevorzugt wird.

Es wird daher postuliert, EPO bei systemischer Applikation in einer subpolyzythämischen wöchentlichen Dosierung von weniger als 90 IU(Internationale Einheiten = Units)/kg Körpergewicht (KGW) zu geben, anstelle von 150 - 300 IU / kg KGW, wie dies für die bekannten EPO-Anwendungen bislang üblich ist. Dadurch soll erreicht werden, dass die Blutbildung im Knochenmarkbereich weniger stimuliert wird, aber nach neuerer Lehre, wie geschildert, die Aktivierung von Endothelzellprogenitoren im Blutbereich möglich ist. Eine Aktivierung der Endothelzellvorläuferzellen im Blut aber auch im Gewebe und die Entwicklung der Endothelzellen, welche die innerste Zellschicht der Blutgefäße bilden, wurde mit einer Verbesserung der Gefäßbildung in Zusammenhang gebracht und es wird vermutet, dass dadurch auch eine Geweberegeneration ermöglicht wird. Dies konnte in der Zwischenzeit in klinischen Versuchen bei Verbrennungswunden belegt werden.

Auch wenn in einigen der genannten Arbeiten eine topische Anwendung von EPO für die Regeneration von Gewebe erwähnt wird, so wird doch die systemische Applikation des Wirkstoffes deutlich in den Vordergrund gestellt, weil auf Grund der Ergebnisse der gefundene nicht-hämatopoietische EPO-Effekt nach Ansicht der neueren Arbeiten primär auf die neu-entdeckte Stimulierung von entsprechenden endothelialen, vaskulären, bzw. CD31-positiven Vorläuferzellen, welche hauptsächlich mit dem Blutstrom zirkulieren, und erst sekundär auf das hierdurch angeregte Wachstum von parenchymen Gewebestrukturen zurückzuführen ist.

Die Verwendung von EPO in einer systemischen Applikation zur Gewebeprotektion oder Geweberegeneration ist jedoch auf Grund des Nebenwirkungspotentials in Bezug auf die hämatopoietischen Effekte mit deutlichen Risiken versehen.

Bei einer topischen Anwendung von EPO, würde, so die bisherige Lehrmeinung, alleine schon aus der unzureichenden Verteilung und Erreichbarkeit der genannten systemisch auftretenden Zellen bzw. Vorläuferzellen durch EPO kein oder nur ein nicht zufriedenstellender Effekt auf die Geweberegeneration zu beobachten sein.

Bei der Regeneration von Geweben nach z.B. Verbrennungstrauma oder Verbrühung oder auch bei ischämischen Wunden ist es notwendig einen raschen Defektverschluss zu erreichen. Dies kann nur geschehen, wenn auch möglichst rasch die Bildung der parenchymatösen Anteile der Haut stimuliert wird. Die zeitlich verschobene Stimulierung einer Komponente (CD 31), um danach die Bildung einer anderen Komponente zu ermöglichen (Parenchym), entspricht der Lehre der Autoren der WO 2005/070450 sowie weitere Veröffentlichungen.

Die Bildung eines Gefäßnetzes kann zwar indirekt unterstützend wirken, stellt aber als solches noch keine Endergebnis dar, da das Parenchym fehlt und unter Umständen erst dann sekundär und damit zeitverzögert gebildet werden kann.

Die Bildung von Gefäßzellen muss also mit einer ortständigen Gewebebildung zeitgleich koordiniert werden. Hierbei entsteht entsprechend der konventionellen Lehre ein Dilemma, da subkutan oder intravenös verbreichte Dosierungen auf Grund eines systemischen Verteilungsprinzips Endothelzellprogenitoren ansprechen und zusätzlich zur Nebenwirkungsbegrenzung subpolyzthämische Dosierungen gegeben werden müssen. Hierbei ist somit nur schwer die Gabe von EPO mit einer örtlich auf den Traumabereich der Haut begrenzten ortständigen Pharmakokinetik verbindbar.

Entsprechend der konventionellen Lehre müssen daher konzeptionelle Stimulierungswege gewählt werden, wobei entweder subpolyzthämische Dosierungen gegeben werden müssen oder wiederholte Injektionen oder auch Veränderungen der Halbwertszeit der Muttersubstanz erforderlich sind.

In jedem Fall aber wird EPO systemisch im Körper verteilt und noch weiter verdünnt am eigentlichen Bedarfsort über die Blutströmung ankommen.

Ferner laufen bei Wunden Vorgänge ab, welche, die topische Anwendung von EPO erschweren: Gewöhnlich läuft die Wundheilung z.B. von verletzter Haut oder Schleimhaut in drei Phasen ab: die Entzündungsphase, die Proliferationsphase und die Wiederaufbau / Remodelling-Phase. Bei einer frischen Wunde oder Hautverletzung, die es zu versorgen gilt, spielen sich innerhalb der ersten 24 Stunden inflammatorische Prozesse ab, welche vor allem das Einwandern diverser Entzündungsfaktoren (wie z.B. Fibronectin) und verschiedenartiger Zellen, wie beispielsweise Monozyten, Phagozyten, polymorphe Zellen und Makrophagen umfassen und schließlich zur Ausbildung einer Fibrinmatrix und vaskulärer Endothelzellen führt. Das dabei entstehende Wundsekret enthält unter anderem eine Reihe von proteolytischen Enzymen sowie in die Wunde eingedrungene Bakterien, die diesbezüglich wirkende Stoffe enthalten.

Die zum Teil hoch aktiven proteolytischen Enzyme sind der Grund, warum auf die Wunde aufgebrachte, die Wundheilung fördernde protein- oder peptidhaltige Arzneimittel, wie EPO, oft wenig oder gar nicht wirksam sind, da das betreffende Protein oder Polypeptid aufgrund seiner chemischen und biologischen Natur durch besagte Enzyme inaktiviert, gespalten und abgebaut wird, ehe es eine ausreichende pharmakologische Wirksamkeit entfalten kann. Durch Infizieren der Wunde mit Bakterien oder Einwandern von Zelldebris wird das Problem zusätzlich verstärkt.

Nicht zuletzt aus diesem Grund werden pharmazeutische Proteine in der Regel systemisch appliziert, wodurch ihre Halbwertszeit beträchtlich verlängert werden kann und sie auch schneller an die Stellen im Körper transportiert werden, an denen sie ihre therapeutische Wirksamkeit entfalten sollen. Allerdings müssen bei dieser Applikationsmethode die Dosen des proteinhaltigen Wirkstoffes ausreichend hoch sein, um den gewünschten therapeutischen Effekt zu erzielen, was oft zwangsläufig zu unerwünschten Nebenreaktionen führt.

Im Falle der therapeutischen Behandlung von Hautverletzungen erscheint überdies eine systemische Applikation eines Wirkstoffes prinzipiell weniger angebracht, da die heilende Wirkung des Arzneimittels eigentlich nur lokal erforderlich ist. Somit besteht ein generelles Problem, wenn zur Behandlung von Hautverletzungen und offenen Fleisch- und Hautwunden proteinhaltige Wirkstoffe eingesetzt werden sollen.

Der Einsatz von Proteinen oder Polypeptiden in topischer Form, wie beispielsweise EPO, das selbst in Plasma eine Halbwertszeit von nur 48 Stunden aufweist, zur Behandlung von derartigen Verletzungen der Haut, wie sie bei übermäßigen mechanischen Einwirkungen und Irritationen sowie bei Verbrennungen und Verbrühungen auftreten können, wäre aber trotz der bekannten Schwierigkeiten sehr wünschenswert.

Es besteht somit die Aufgabe, EPO oder seine bioäquivalenten Derivate, Fragmente, Mimetika und dergleichen für die Wundheilung der Haut in Form einer topischen Anwendung bereit zu stellen, ohne dass es einerseits, wie soeben beschrieben, zu dramatischen Wirkungsverlusten durch Proteolyse von EPO auf Grund enzymatischer oder anderer Vorgänge in der Wunde kommt, andrerseits aber, wie ebenfalls oben ausführlich erläutert, eine Stimulierung von zur Wundheilung befähigten Zellen oder Vorläuferzellen im unmittelbaren Wundbereich und nach Möglichkeit auch in tieferen Gewebeschichten im Umfeld der Wunde durch topisch appliziertes EPO ermöglicht werden kann, was letztlich zu einem rascheren Wundverschluss führen sollte und eine topische Anwendung von EPO erst sinnvoll erscheinen lässt und ihr einen deutlichen Vorteil gegenüber der systemischen Anwendung einräumt.

Ziel der Erfindung ist es ferner eine Verabreichungsform zu schaffen, die sich nicht an den Risiken und Beschränkungen einer systemischen, insbesondere subpolyzthämischen Dosierung von EPO orientieren muss, und zugleich spezifisch auf die eigentlichen gewebsrelevanten Zellen wirken kann, ohne die ortsständigen adulten Endothelzellen dabei auszuschließen.

### ZUSAMMENFASSUNG DER ERFINDUNG:

Es wurde nun überraschenderweise gefunden, dass EPO bei topischer Anwendung vorzugsweise in einer geeigneten, den Wirkstoff stabilisierenden und ihn gleichmäßig abgebenden Formulierung bei Verletzungen oder pathologisch bedingten Schäden der Haut wirksam ist, wobei diese Wirksamkeit, wie insbesondere auch histologische Untersuchungen zeigen, nicht oder kaum auf die bekannte prognostizierte Aktivierung endothelialer und anderer Vorläuferzellen, welche im Blut zirkulieren und zur Wunde transportiert werden können, zurückzuführen ist - diese wäre für den anzustrebenden Effekt nicht ausreichend - , sondern offensichtlich auf durch EPO bedingte Stimulierung von Zellen, welche ortsständig also im unmittelbaren Wundbereich oder im erweiterten Umfeld der Wunde angesiedelt sind, beruht, wobei überraschenderweise vorwiegend solche Zellen im Hautgewebe funktionell zur Wundheilung beitragen, die parenchymatös sind, also keine Gefäßzellen oder deren Vorläuferzellen und auch vorwiegend keine im Wundgewebe befindlichen Endothelzellen oder deren Vorläuferzellen sind.

Es wurde generell gefunden, dass insbesondere solche Zellen im Wundbereich, die keine oder nur geringfügig den Endothelzell-Oberflächenmarker CD31 tragen bzw. exprimieren, durch topisch appliziertes EPO direkt und lokal zu Differenzierung und Wachstum angeregt werden.

CD31 wird üblicherweise auf Endothelzellen bzw. Gefäßzellen, Platelets, Makrophagen Granulozyten, T-Zellen, NK-Zellen, Lymphozyten und Fibroblasten gefunden. CD31 exprimierende Zellen gelten als Neo-Angiogensefaktoren. In Geweben stellen die Gefäße Strukturen dar, die das Gewebe wie Flüsse durchdringen, in keiner Weise jedoch das Gewebe selbst darstellen. Diese Gefäßzellen und deren Vorläuferzellen haben Oberflächenmarker wie CD31, wodurch diese von anderen Zelltypen exakt unterscheidbar und abgrenzbar sind. Parenchymatöse Anteile der Haut stellen CD31 negative Zelltypen wie Keratinozyten, Haarwurzelzellen sowie dermale Zellen wie Bindegewebszellen dar. Vorzugsweise werden also CD31-negative Zellen, insbesondere Keratinozyten, Haarwurzelzellen und Bindegewebszellen oder Zellen, die CD31 nur geringfügig exprimieren, durch lokal appliziertes EPO unmittelbar und vor Ort stimuliert. Dieser weitere Wirkeffekt ist neu und unterscheidet sich sowohl von dem bekannten hämatopoietischen EPO-Effekt als auch von dem nicht-hämatopoietischen vor kurzem aufgefundenen und systemisch nachgewiesen Mechanismus (siehe oben).

Es ist in diesem Zusammenhang hervorzuheben, dass insbesondere die systemische Applikation von Erythropoetin und deren Derivate und Analoge bei einer subkutanen oder intravenösen Gabe nur einen unzureichenden oder zu kurzen Effekt an dem Ort der Verletzung der Haut bewirkt, zumal dieses systemische Konzept die Bedeutung der gewebespezifischen Stammzellen, die völlig unabhängig von Gefäßzellen sind, vernachlässigt.

Erfindungsgemäß können also erstmals die ortständigen Vorläuferzellen in Hautanhangsgebilden und den Stammzellkrypten der Haut durch topische EPO-Gabe direkt angeregt werden. Ferner wurde festgestellt, dass über die durch EPO bedingte Stimulierung der besagten weitgehend CD31-negativen Zellen auch CD90 positive Zellen und Nestinpositive Zellen im Wundbereich der Haut angeregt werden. CD90 ist ein Markerprotein für Stammzellen und neuronale Vorläuferzellen, während Nestin ein Marker für Nervenzellen ist. Auch CD73-positive Zellen im Wundbereich werden direkt durch EPO mitstimuliert. Damit vermag topisch appliziertes EPO im unmittelbaren Wundbereich nahezu alle wichtigen Zellen zum Wachstum anzuregen, welche für die Wundheilung der Haut essentiell sind.

Erfindungsgemäß wird also völlig unabhängig von den Vorgaben und Einschränkungen subpolzythämischer Dosierungsanforderungen des Standes der Technik bei der Verwendung von topisch appliziertem EPO vorzugsweise in einer geeigneten Formulierung auf Basis von Polymeren, insbesondere hydrophiler Polymere, und seinen wirkungsgleichen oder wirkungsähnlichen Derivaten und Analoga eine direkte Stimulierung von essentiellen wundheilungsspezifischen Zellen und Vorläuferzellen in der Haut herbeigeführt. Dies geschieht unabhängig von einer Einwirkung auf Endothelzellprogenitoren im Gesamtorganismus, welche notgedrungen bei einer konventionellen systemischen Applikationsform erfolgt. Eine solche systemische Wirkung ist in der Regel wegen der eintretenden hämatopoietischen Effekte (Erhöhung der Anzahl der roten Blutzellen, gesteigerte Blutbildung im Knochenmark, erhöhtes Thromboserisiko etc.) unerwünscht.

Topisch lokal angewendetes EPO gemäß der Erfindung bewirkt ferner keine oder weitgehend keine Stimulierung systemisch im Blutstrom zirkulierender vaskularer oder endothelialer Vorläuferzellen. Die direkte Stimulierung der wundnahen Zellen hat den Vorteil, dass das regionale Haut- und Wundmilieu einerseits die systemische Resorption durch z.B. Proteasen begrenzt, die den Abbau von EPO vor Eintritt in den systemischen Zirkulationsbereich reduziert und trotzdem eine ortständig hohe Wirkstoffkonzentration ermöglicht. EPO-Mengen, die bei einer konventionellen systemischen Applikationsform eine Konzentration von 250 IU EPO / KGW und mehr bedeuten würden, können ohne weiteres verabreicht werden, ohne dass die entsprechenden genannten unerwünschten EPO-Wirkungen eintreten. Hierdurch wird die therapeutische Breite für den Bereich der Geweberegeneration erheblich erweitert. Zusätzlich können co-stimulatorische Effekte durch die nur ortständig in hohen Konzentrationen vorhanden Traumacytokine durch die topische EPO-Gabe initiiert werden.

Gegenstand der Erfindung ist somit:
- die Verwendung von Erythropoietin oder eines seiner biologisch wirkungsgleichen Derivate oder Analoga (EPO) vorzugsweise in Form einer den Wirkstoff stabilisierenden und ihn gleichmäßig freisetzenden pharmazeutischen Formulierung auf Basis vorzugsweise von Polymeren mit zumindest teilweise hydrophilen Eigenschaften zur Herstellung eines topisch lokal anzuwendenden Medikamentes zum Defektverschluss und / oder zur Reepithelialisierung von traumatisierter Haut durch gezielten Stimulierung von gewebespezifischen Vorläuferzellen, die keine vaskulären Vorläuferzellen sind und von adulten gewebespezifischen Zellen, die keine Endothelzellen sind, , und die jeweils ortspezifisch im unmittelbaren Wundbereich der traumatisierten Haut angesiedelt sind.
- eine entsprechende Verwendung, wobei das topisch lokal angewendete EPO keine oder weitgehend keine Stimulierung systemisch im Blutstrom zirkulierender vaskularer oder endothelialer Vorläuferzellen bewirkt.
- eine entsprechende Verwendung, wobei die besagten durch topisch angewendetes EPO stimulierte Zellen kein oder nur geringfügig CD31 Antigen exprimieren.
- eine entsprechende Verwendung, wobei die Reepithelialisierung der Haut 20 bis 70 %, vorzugsweise 20 bis 50%, insbesondere 30 - 40%, schneller erfolgt als durch EPO in einer nicht stabilisierten Form unter sonst gleichen Bedingungen.
- eine entsprechende Verwendung, wobei die Reepithelialisierung der Haut 20 bis 50 %, vorzugsweise 30 bis 40% schneller erfolgt als durch eine entsprechende pharmazeutische Formulierung ohne EPO unter sonst gleichen Bedingungen.
- eine entsprechende Verwendung, wobei topisch lokal angewendetes EPO die Differenzierung und das Wachstum von Keratinozyten und/oder Haarwurzelzellen und/oder Bindegewebszellen stimuliert.
- eine entsprechende Verwendung, wobei das topisch lokal angewendetes EPO zusätzlich die Differenzierung und das Wachstum von CD90-positiven und/oder Nestin-positiven Zellen im unmittelbaren Wundbereich stimuliert.
- eine entsprechende Verwendung, wobei das topisch lokal angewendete EPO die Differenzierung und das Wachstum von Nervenzellen und / oder neuronaler Vorläuferzellen anregt.
- eine entsprechende Verwendung, wobei entsprechende Vorläuferzellen in tieferen Gewebeschichten im lokalen Umfeld der Wunde durch das topisch lokal angewendete EPO stimuliert werden.

Eine der wesentlichen Probleme bei der systemischen Gabe von EPO ist es, unter Berücksichtigung der systemischen Nebenwirkungen einerseits und der Notwendigkeit der Einstellung einer hohen Wirkstoffkombination andrerseits, in regionalen Bereichen eine ausreichend hohe gewebeprotektive Wirkung zu erzielen.

Ziel der Erfindung war es daher auch, ein Applikationsverfahren und ein Trägermaterial zu schaffen, mit dem EPO und dessen Analoga besonders effektiv topisch angewendet werden kann.

Überraschenderweise wurde gefunden, dass der beschriebene Wirkeffekt von topisch appliziertem EPO und seiner biologisch wirkungsgleichen Derivate und Analoga besonders ausgeprägt ist, wenn der Wirkstoff in einer ihn stabilisierenden pharmazeutischen Zubereitung oder Formulierung zur topischen Applikation angeboten wird, wobei die Formulierung bzw.

Zubereitung die weitere Eigenschaft aufweisen sollte, dass der Wirkstoff möglichst gleichmäßig und relative langsam an die Wunde abgegeben werden kann (Slow-Release).

Es wurde überraschenderweise gefunden, dass gelbildende hydrophile, höher viskose Polymere wie Cellulosederivate, Carbomere, Fettalkohole oder Macrogole (Polyethylenglykole) oder Gemische von diesen, vorzugsweise gelbildende Polysaccharide, insbesondere aus der Gruppe der Celluloseether und Celluloseester, als Trägerstoff oder Formulierungsgrundlage eine stabilisierende Wirkung auf EPO und seine Derivate in entsprechenden topisch anzuwendenden EPO haltigen Zubereitungen, wie Salben, Cremes, Pasten oder Gelen ausüben. Hierbei befindet sich der Wirkstoff gleichmäßig verteilt in einer viskosen, gequollenen, polymerisierten oder gelartigen Matrix, vorzugsweise Polysaccharid-Matrix, die ihn schützt, und aus welcher er zudem gleichmäßig und langsam an die Wunde abgegeben wird, in der er direkt und unmittelbar, ohne dass eine nennenswerte Zersetzung durch proteolytische Enzyme stattfindet, seine Wirkung entfalten kann.

Dabei zeigen interessanterweise EPO - haltige Gele mit relativ hoher Viskosität von mehr als 20.000 mPa s, vorzugsweise größer als 30.000 mPa s, insbesondere zwischen 20.000 und 100.000 mPa s, vorzugsweise zwischen 40.000 und 60.000 mPa s vorteilhaftere Ergebnisse als weniger viskose Gele mit Viskositätswerten unter 20.000 mPa s, insbesondere unter 10.000 mPa s. Solche Viskositätswerte können in Gelen erzielt werden, bei denen das gelbildende Polymer, vorzugsweise der Polysaccharid-Gelbildner einen Anteil von 2 - 4 Gew. %, vorzugsweise 2 - 3% besitzt, jedoch nicht deutlich weniger.

Als besonders vorteilhaft haben sich Gel-Formulierungen erwiesen, die als Gelbildner mindestens ein quellbares Polysaccharid, ausgewählt aus der Gruppe bestehend aus Hydroxmethylcellulose, Hydroxyethycellulose, Carboxymethylcellulose und Carbooxyethylcellulose enthalten. Bevorzugt sind solche Formulierungen, die Carboxmethylcellulose und / oder Hydroxethylcellulose enthalten oder aus diesen bestehen.

Neben gelartigen Zusammensetzungen auf Basis von Polysacchariden, wie Cellulosen oder auch Alginaten, sind prinzipiell auch andere quellbare Polymere mit hydrophiler Gesamtwirkung und den erforderlichen viskosen Eigenschaften geeignet, wie sie in vielen im Markt befindlichen Formulierungen, z.B. Gelen, Pasten, Salben, verwendet werden.

Auch können hydrophobe polymere in der Regel hochviskose Matrizes, beispielsweise auf Basis von Polyacrylaten oder Polyurethanen, verwendet werden, in denen hydrokolloidale Partikel aus Polysacchariden wie Carboxymethyl/ethylcellulosen, Gelatine oder Pektine, die den Wirkstoff enthalten, eingelagert sind.

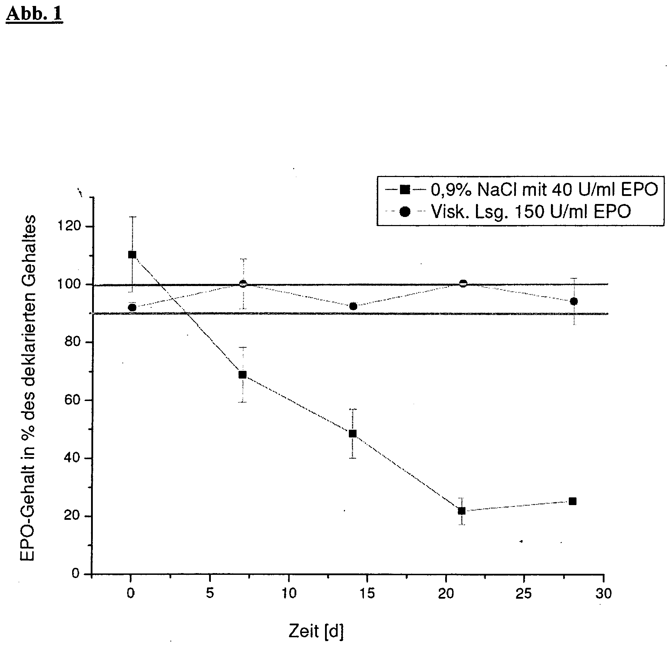

Das EPO in den erfindungsgemäßen vorteilhaften Gel-Formulierungen zeigt sich über einen langen Zeitraum von mehr als 30 Tagen stabil (Abb. 1), während der EPO-Gehalt in einer vergleichbar konzentrierten wässrigen EPO Lösung (in 0.9%iger NaCl) bereits nach 20 Tagen um 80% abgenommen hat.

Demgegenüber heilt eine Wunde, welche mit einer EPO-haltigen erfindungsgemäßen Formulierung behandelt wurde, im Vergleich zu einer erfindungsgemäßen Formulierung ohne EPO um etwa 10 - 50%, vorzugsweise 20 - 40% schneller. So kann beispielsweise eine Wundheilung durch Reepithelialisierung nach 4 bis 8 Tagen mit einer EPO-haltigen erfindungsgemäßen Formulierung zwischen 92 und 99% erreicht werden, während unter sonst gleichen Bedingungen die Wundheilung mit der erfindungsgemäßen Formulierung ohne EPO nur maximal bis zu 85 - 87% erfolgt.
Gegenstand der Erfindung ist somit:
- eine gelartige oder viskose Formulierung zur topischen Anwendung bei der Behandlung von Wunden, von Verbrennungen oder Verbrühungen der Haut oder von Hauterkrankungen mit begleitenden chronischen Wunden, umfassende Erythropoietin (EPO) und mindestens ein gelbildendes quellfähiges Polysaccharid in einer Konzentration von 2-4 Gew. %, ausgewählt aus der Gruppe bestehend aus
   - Hydroxyethylcellulose
   - Hydroxymethylcellulose
   - Carboxymethylcellulose wobei die gelartige oder viskose Formulierung im gequollenen Zustand
      (i) eine Viskosität von 20.000-60.000 mPa x s aufweist,
      (ii) EPO in einer Konzentration von 100-500 IU/g enthält,
      (iii) in einer Menge auf die traumatisierte Haut aufgetragen wird, die 50-1.5000IU, EPO/cm Wundfläche entspricht, und
      (iv) EPO in der Formulierung stabilisiert und gleichmässig und langsam freisetzt.
      Des Weiteren hierin offenbart sind:
      - eine viskose Formulierung auf Basis mindestens eines gelbildenden hydrophilen Polymers mit einer Viskosität von mindestens 20.000 mPA x s, welches Erythropoietin oder eines seiner biologisch wirkungsgleichen Derivate oder Analoga (EPO) enthält, und dieses gleichmäßig an eine hydrophile Umgebung abzugeben vermag.
- eine entsprechende viskose Formulierung, welche eine Viskosität von mindestens 30.000 mPA x s, vorzugsweise 40.000 bis 60.000 mPA x s aufweist.
- eine entsprechende viskose Formulierung, welche als hydrophiles Polymer vorzugsweise ein Celulosederivat, insbesondere ein Polysaccharid ausgewählt aus der Gruppe der Hydroxyalkylcellulosen und / oder Caboxyalkylcellulosen, sowie eine hydrophobe Polymermatrix auf Basis von Polyacrylat oder Polyurethan.
- eine gelartige oder viskose Formulierung auf Basis mindestens eines quellfähigen Polysaccharides, ausgewählt aus der Gruppe der Hydroxyalkylcellulosen und / oder Caboxyalkylcellulosen, welche Erythropoietin oder eines seiner biologisch wirkungsgleichen Derivate oder Analoga (EPO) enthält.
- eine entsprechende Formulierung, die durch Vermischen von EPO in lyophilisierter gelöster oder suspendierter Form mit dem vorgequollenen Polysaccharid erhältlich ist.
- eine entsprechende Formulierung, bei der das fertig gequollene Polysaccharid eine Viskosität von 5.000 - 100.000 mPa x s, insbesondere 20.000 - 50.000 mPa x s aufweist.
- eine entsprechende Formulierung, bei der das oder die Polysaccharide in einer Konzentration von 0.4 bis 4 Gew. %, insbesondere 2 - 3 Gew. % eingesetzt werden.
- eine entsprechende Formulierung, bei der das Polysaccharid ein Celluloseether und / oder ein Celluloseester ist und ausgewählt wird aus einem oder mehreren Mitgliedern der Gruppe bestehend aus:
   -- Hydroxyethylcellulose
   -- Hydroxymethylcellulose
   -- Carboxyethylcellulose
   -- Carboxymethylcellulose
- eine bevorzugte Formulierung enthaltend als Gelbildner polymerisierbare Hydroxyethylcellulose und / oder Carboxymethylcellulose.
- eine entsprechende Formulierung, welche EPO in einer Konzentration von 100 bis 500 IU/g Gel-Formulierung, insbesondere 150 bis 300 IU/g Gel-Formulierung, vorzugsweise 150 IU /g Gel-Formulierung enthält.
- eine entsprechende Formulierung, welche 0.5g bis 5g Gel-Formulierung/cm² Wundfläche, vorzugsweise 1g bis 3g Gel-Formulierung /cm² Wundfläche, insbesondere 1.0g / cm² Wundfläche enthält.
- eine entsprechende Formulierung, welche je nach verwendete Gel-Menge eine Menge an EPO aufweist, die bei einer Wundfläche von etwa 100 cm² einer systemischen Gabe von etwa 50 - 2.200 IU / kg Körpergewicht (KGW, bei 70 kg Standardgewicht) entspräche, bzw. etwa 100 - 4.500 IU / KGW für eine Wundfläche von etwa 200 cm² , und etwa 150 - 6.000 IU / KGW für eine Wundfläche von etwa 300 cm² .
- eine entsprechende Formulierung, welche je nach Wundgröße bzw. Wundfläche (1 cm² bis 300 cm²) eine EPO Menge von etwa 50 bis etwa 450.000 IU, durchschnittlich etwa 500 bis 300.000 IU enthält, vorzugsweise 1.500 bis 60.000 IU, insbesondere 3.000 bis 10.000 IU.
- eine entsprechende Formulierung, welche zusätzlich mindestens einen weiteren aktiven Wirkstoff enthält, der vorzugsweise antibakteriell, antiviral, fungizid, oder antiinflammatorisch , als Proteinaseinhibitor wirkt oder der Wundheilung anderweitig förderlich ist
- eine entsprechende Formulierung, welche zusätzlich mindestens ein synthetisches Copolymer enthält.
- eine entsprechende Formulierung, welche zusätzlich einen oder mehrere Hilfsstoffe enthält.
- eine entsprechende Formulierung, welche in oder auf eine feste Trägermatrix eingebracht
- ist, welche die Wirkstoffabgabe moduliert, und insbesondere das EPO gleichmäßig in den Wundbereich der traumatisierten Haut abgibt.
- eine entsprechende Formulierung, bei der die feste Trägermatrix ein Pflaster, eine Folie, ein Film, ein Verband, eine Gaze, insbesondere aber ein dreidimensional strukturiertes Pflaster ist, welches in der Lage ist, die Formulierung selbst zu stabilisieren und zu schützen.
- ein pharmazeutischer Kit-of-Parts, umfassend mindestens zwei getrennte Packungs-Einheiten, wobei die erste Einheit mindestens ein quellfähiges Polymer enthält und die zweite Einheit lyophilisiertes EPO oder ein entsprechendes Derivat davon enthält.

Die erfindungsgemäßen Formulierungen können zur topischen und lokalen Behandlung von verletzter Haut sowie unterschiedlich verursachten Erkrankungen der Haut bei Mensch und Tier verwendet werden. Die erfindungsgemäßen Formulierungen können insbesondere auch in der Zahnmedizin und/oder zur Verbesserung der Wundheilung in der Allgemeinmedizin oder Tiermedizin verwendet werden. Die erfindungsgemäßen Formulierungen können auch bei topischen und lokalen Behandlungen von postoperativen oder posttraumatischen Wundinfektionen und Verbrennungen, bei postoperativen Sepsis, bei endzündeten und / oder infizierten Ulzera, bei chronischen ischämischen Wunden, bei akuten und chronischen Hautinfektionen oder Dermatosen, bei Akne, Rosazea, Psoriasis oder bei Schleimhautulzerationen, oder Schleimhautverletzungen, wie Schleimhaut-Knochenwunden, insbesondere im Kiefer- bzw. Dentalbereich.

Gegenstand der Erfindung ist somit auch die Verwendung der oben und unten spezifizierten Formulierung zur Herstellung eines Arzneimittels zur topischen und lokalen Behandlung und Heilung von Wunden durch Reepithelialisierung der Haut, insbesondere zur Behandlung und Heilung von Verbrennungswunden oder von Haut-, Schleimhaut und Knochenwunden und Wunden im Dentalbereich.

### KURZE BESCHREIBUNG DER ABBILDUNGEN:

### Abb. 1: Stabilitätsstudie von EPO-haltigen Zubereitungen

Wie Abb. 1 entnommen werden kann, bewegt sich der EPO-Gehalt während der Untersuchung im Bereich von 90 - 100 % des Nenngehaltes (150 U/ml), so dass von der Stabilität der Zubereitung und auch des entsprechenden Hydrogels über einen Zeitraum von 4 Wochen nach Herstellung auszugehen ist.

Im Gegensatz dazu zeigte sich in der kochsalzhaltigen Lösung (0.9 Gew. % NaCl in Wasser mit 40 U/ml EPO) über den Versuchszeitraum hinweg ein deutlicher Abbau von EPO, so dass nach drei und vier Wochen nur noch zwischen 20 und 30 % des Nenngehaltes festgestellt werden konnten.

### Abb. 2:

Die Abbildung gibt die Reepithelialisierung der Haut von traumatisierten Ratten in % wieder, die mit einer erfindungsgenäßen Formulierung (Carboxymethylcellulose 3%) ohne (Kontrolle/Placebo, Gel F linker Balken) und mit EPO (ca. 0.3g Gel: 150 IU / g Gel = etwa 200 IU / KGW, Gel E - rechter Balken) 4 Tage behandelt wurden..

Die Ergebnisse sind in Tabellenform (oberes Bild) und graphisch (unteres Bild) dargestellt.

### Abb. 3:

Die Abbildung gibt die Reepithelialisierung der Haut von traumatisierten Ratten in % wieder, die mit einer erfindungsgemäßen Formulierung (Carboxymethylcellulose 3%) ohne (Kontrolle/Placebo, Gel F- linker Balken) und mit EPO (ca. 0.3g Gel: 150 IU / g Gel = etwa 200 IU / KGW, Gel E - rechter Balken) 8 Tagen behandelt wurden. Der Verband / Pflaster wurde nach vier Tagen gewechselt und die Wunde mit frischem Gel behandelt. Nach 8 Tagen Behandlung mit der erfindungsgemäßen Formulierung ist die Haut nahezu zu 100 % wiederhergestellt.

Die Ergebnisse sind in Tabellenform (oberes Bild) und graphisch (unteres Bild) dargestellt.

### Abb. 4:

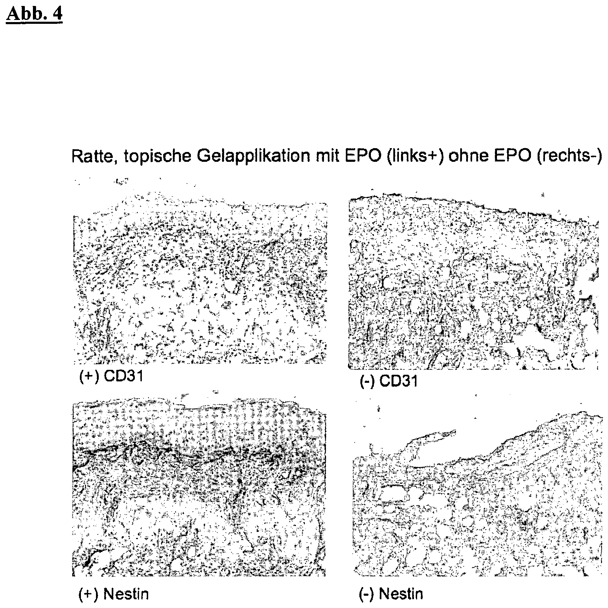

Abbildung 4 stellt die histologische Untersuchung der Wundheilung nach 8 Tagen Behandlung mit einem Gel wie in Beispiel 8 beschrieben dar. Dabei wurden durch entsprechende Einfärbung Zellen dargestellt, die CD31 Protein und Nestin exprimieren.

Die Ergebnisse zeigen, dass bei der Behandlung mit EPO die Schichtdicke des Epithels deutlich erhöht ist gegenüber der Kontrollformulierung. Überdies kann gesehen werden, dass die Neubildung des Epithels im wesentlichen nicht korreliert ist mit einer Zunahme an CD31 positiven (eingefärbten) Zellen, wohl aber zu einem gewissen Grad mit Nestin-positiven Zellen.

### Abb. 5:

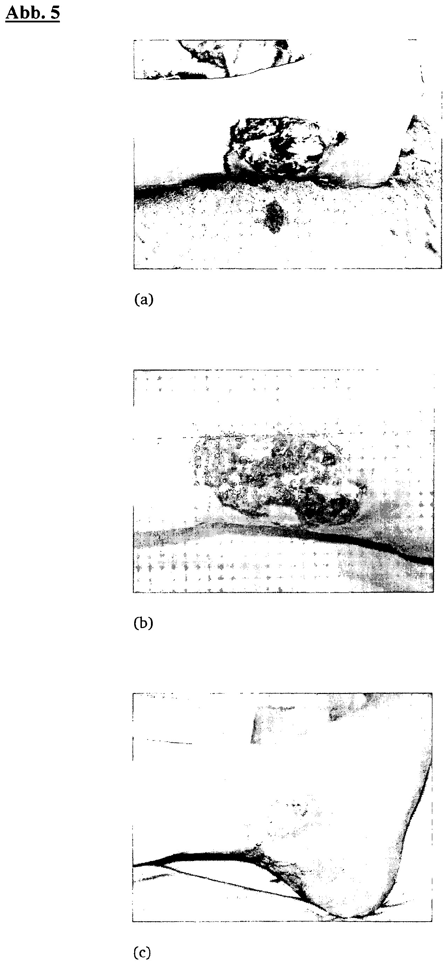

Abbildung 5 ist der fotographische Nachweis der Heilung eines chronisch ischämischen Ulcus malleolaris lateralis dritten Grades eines diabetischen Patienten nach dreimaliger lokaler Behandlung mit der erfindungsgemäßen Formulierung enhaltend 3.000 IU EPO (insgesamt 9.000 IU). Das untere Bild stellt den Verlauf nach 15 Tagen dar (Beispiel 16).

### Abb. 6:

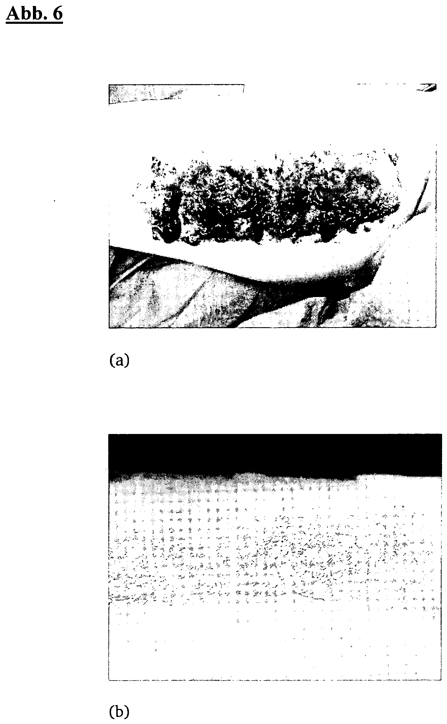

Abbildung 6 zeigt fotographisch den Heilungsverlauf einer thermischen Wunde nach Spalthautentnahme bei einem Patienten durch Behandlung mit einmaliger Gabe von 3.000 IU EPO eingebracht in einem erfindungsgemäßen Hydrogel (Beispiel 17).

### EINZELHEITEN DER ERFINDUNG

Es konnte festgestellt werden, dass durch topisch appliziertes EPO, insbesondere in Form einer oben und unten näher beschriebenen gelartigen Formulierung, insbesondere eines Hydrogels auf Cellulose-Basis, die Hautregeneration z.B. nach Verbrennungstrauma, Verbrühung, oder krankheitsbedingte Wunden ein rascher Defektverschluss erzielt werden kann. Ferner wird durch topisch verabreichtes EPO die Reepithelialisierung des Wundbereiches entscheidend gefördert. Dies ist erkennbar an einer höheren Schichtdicke des Epithels zu erreichen (Abb. 4). Dies kann nur dann geschehen, wenn auch möglichst rasch die Bildung der parenchymatösen Anteile der Haut stimuliert wird.

Ferner kann beobachtet werden, dass auch die Ausbildung eines Gefäßnetzes durch topisch appliziertes EPO initiiert wird, wobei dies allerdings mit der lokalen Ausbildung des Parenchyms einhergeht, im Gegensatz zur bekannten Lehre, dass die durch EPO bedingte Wundheilung zunächst durch Stimulierung von Gefäß bildenden Zellen in Gang gesetzt wird, also EPO lediglich die Gefäßbildung bewirkt und nicht direkt und bestenfalls zeitversetzt die Bildung von parenchymatösen Gewebe einsetzt, als Folge der EPO -Stimulierung von Endothelzellen und deren Vorläuferzellen.

Die Ergebnisse der vorliegenden Erfindung zeigen aber klar, dass die Bildung von Gefäßzellen mit einer ortständigen Gewebebildung mindestens zeitgleich koordiniert wird.

Erfindungsgemäß kann ferner festgestellt werden, dass durch die topische Applikation von EPO gewebespezifische Vorläuferzellen im Grenzbereich zur neuen Epidermis verstärkt zu finden sind, welche Nestin-positiv sind. Somit werden also neuronale Stammzellen in diesem Bereich stimuliert, was die verbesserte Sensibilität der Haut bei der Wundheilung erklärt.

Für die erfindungsgemäßen Formulierungen wird vorzugsweise rekombinantes Erythropoietin, so wie es im Handel erhältlich ist, eingesetzt. Es können aber erfindungsgemäß auch EPO-Derivate eingesetzt werden, welche entwickelt worden sind, um die Halbwertszeit des Wirkstoffes im Blut bzw. im Serum gegenüber dem nativen EPO zu verlängern. Solche EPO-Derivate können auch für die topische Anwendung eingesetzt werden, obwohl dies nicht zwingend erforderlich ist, weil die erfindungsgemäßen gelartigen Formulierungen eine eigene spezielle Schutzwirkung gegenüber dem normalen EPO entfalten. Hierzu zählen z.B. pegyliertes EPO, in seinem Glycosylierungsmuster verändertes EPO (z.B. Aranesp®), sialyliertes EPO, oder EPO, welches mit anderen Polypeptiden oder Fragmenten von Immunglobulinen (z.B. mit dem Fc-Teil eines Antikörpers) fusioniert wurde (bekannt z. B. aus WO 02/49673 oder WO 01/02017). Ferner, können auch biologisch wirksame synthetische EPO Peptid-Mimetika (wie bekannt, z. B. aus WO 96/40749, WO 96/40772, WO 01/38342, WO 01 091780, WO 2004/101611; WO 2004/100997, WO 2004/101600, WO 2004/101606 und WO 2006/050959) eingesetzt werden. Da diese Mimetika meist deutlich kürzer in ihrer Aminosäurekette sind und damit in der Regel einem schnelleren Abbau unterliegen, erfahren sie durch die erfindungsgemäßen Formulierungen die notwendige und ausreichende Stabilisierung.

Bei der bekannten systemischen Anwendung von EPO, wird der Wirkstoff in der Regel zwischen 150 und 300 IU / kg Körpergewicht (KGW) pro Dosis appliziert. Bei deutlich höheren Dosen werden die unerwünschten Nebenwirkungen dominant. Bei einem erwachsenen Menschen (70 kg) entspricht dies zwischen 10.500 und 31.500 IU EPO / Dosis. Dies entspricht wiederum etwa 80 bis 250µg EPO / Dosis (13.000 IU ∼ 100µg EPO).

Bei der erfindungsgemäßen topischen Anwendung bei Verletzungen der Haut mittels der erfindungsgemäßen Formulierungen bzw. Gele können deutlich höhere EPO -Mengen pro Dosis eingesetzt werden, ohne dass nennenswerte Nebeneffekte auftreten. So können bei entsprechend großen Wunden (z. B. 300 cm²) EPO-Mengen eingesetzt werden, die ca. 6.500 IU / kg Körpergewicht (KGW) bei systemischer Gabe entsprechen würden. Eine solche Menge EPO wäre systemisch appliziert, nicht mehr tolerabel. Je nach Wundgröße und Menge Gel für eine Wundfläche und Gehalt an EPO / g Gel, variieren die benötigten Gesamtmengen an EPO erheblich.

In der Regel kann man erfindungsgemäß als Dosis die Menge an EPO ansehen, welche für die einmalige Behandlung einer (unterschiedlich großen) Wunde benötigt wird. Da eine Wunde nicht nur durch ihre Fläche, sondern auch gegebenenfalls durch ihre Tiefe definiert ist, kann die Dosis an notwendigen Wirkstoff stark variieren, da die Wunde vorzugsweise mit der erfindungsgemäßen Formulierung ausgefüllt wird.

Setzt man 70 Kg als Körpergewicht für einen erwachsenen Menschen an, so ergeben sich für die Wundheilung beispielsweise folgende Werte:
50 - 500 IU / KGW → 3.500 - 35.000 IU EPO pro Dosis bzw. Wunde und Wundgröße;
50 - 150 IU / KGW → 3.500 - 10.500 IU EPO pro Dosis bzw. Wunde und Wundgröße;
100 - 500 IU / KGW → 7.500 - 35.000 IU EPO pro Dosis bzw. Wunde und Wundgröße;
150 - 300 IU / KGW → 10.500 - 21.000 IU EPO pro Dosis bzw. Wunde und Wundgröße.
Aus der Tabelle 1 ist zu entnehmen, welchen Wundgrößen und Gelkonzentrationen diese Werte entsprechen.

Die niedrigen EPO-Einheiten werden vorzugsweise für kleiner Wunden mit einer Wundfläche von etwa 5 - 20 cm² eingesetzt, während die oberen Werte für große Wunden mit einer Fläche von um 100 cm² und größer Anwendung finden. Bei Wunden mit eine Fläche von beispielsweise 10 cm² sind erfindungsgemäß EPO-Mengen zwischen etwa 500 und 15.000 IU erfindungsgemäß einsetzbar. Bei Wunden mit einer Fläche von beispielsweise 100 cm² werden etwa 5.000 - 150.000 IU EPO benötigt. Dies bedeutet, dass durchschnittlich etwa 50 - 1.500 IU EPO / cm² Wundfläche, vorzugsweise 75 - 450 IU EPO / cm² zur Reepithelialisierung und Defektverschluss der Wunde erfindungsgemäß auf die Wunde aufgetragen werden sollten. Selbstverständlich können die genannten Werte nach oben und unten unter bestimmten Bedingungen deutlich über- bzw. unterschritten werden.

Den oben genannten Werten liegt ein EPO-Gehalt von 100 - 500 IU / g Gel-Formulierung, insbesondere 100 -300 IU / g Gel-Formulierung, vorzugsweise 150 - 200 IU / g Gel-Formulierung zugrunde. Im einzelnen werden erfindungsgemäß folgende Bereiche von EPO -Mengen pro Gramm Formulierungsgrundlage eingesetzt:
100 IU / g Gel - 300 IU / g Gel, 100 IU / g Gel - 150 IU / g Gel,
100 IU / g Gel - 200 IU / g Gel, 100 IU / g Gel - 250 IU / g Gel,
150 IU / g Gel - 200 IU / g Gel, 150 IU / g Gel - 250 IU / g Gel,
150 IU / g Gel - 300 IU / g Gel, 200 IU / g Gel - 250 IU / g Gel,
250 IU / g Gel - 300 IU / g Gel,

In der Regel werden je nach Verletzung bei einem Erwachsenen etwa 0.5 bis etwa 5g, vorzugsweise 1 - 3g, insbesondere 1g erfindungsgemäßes Gel pro Quadratzentimeter (cm²) Wundfläche verwendet. Diese Konzentrationen können aber nach beiden Seiten hin unter- bzw. überschritten werden. Insbesondere werden folgende Gel-Konzentrationsbereiche eingesetzt: 0.5g Gel/ cm² - 2.0g Gel/cm², 0.5g Gel/ cm² - 1.0g Gel/ cm², 1.0g Gel / cm² - 2.0g Gel / cm².

Erfindungsgemäße Formulierungen enthalten somit pro Dosis entsprechend der Wundgröße und der EPO-Menge pro Gramm Formulierungsgrundlage beispielsweise etwa die folgenden Mengen an EPO (IU) oder die Mengen an EPO, die in Tabelle 1 angegeben sind: 2.000 IU, 3.000 IU, 4.000 IU, 5.000 IU, 6.000 IU, 7.000 IU, 8.000 IU, 9.000 IU, 10.000 IU, 12.000 IU, 15.000 IU, 20.000 IU, 25.000 IU, 30.000 IU, 35.000 IU, 40.000 IU, 45.000 IU, 55.000 IU, 60.000 IU, 70.000 IU, 80.000 IU , 90.000 IU, 100.000 IU, 120.000 IU, 135.000 IU, 150.000 IU und 180.000 IU.

**Tabelle 1:**

| (A) 0.5g Gel / cm² Wundfläche | | | | | | |
|---|---|---|---|---|---|---|
| | | **IU EPO** | **IU EPO** | **IU EPO** | **IU EPO** | **IU EPO** |
| **cm² Wundfläche** | Menge Gel (g) | (100 IU /g Gel) | (150 IU/g Gel) | (200 IU/g Gel) | (300 IU/g Gel) | (500 IU/g Gel) |
| **1** | **0.5** | **50** | **75** | **100** | **150** | **250** |
| 10 | 5 | 500 | 750 | 1.000 | 1.500 | 2.500 |
| 100 | 50 | 5.000 | 7.500 | 10.000 | 15.000 | 25.000 |
| 200 | 100 | 10.000 | 15.000 | 20.000 | 30.000 | 50.000 |
| 300 | 150 | 15.000 | 22.500 | 30.000 | 45.000 | 75.000 |

| (B) 1g Gel / cm² Wundfläche | | | | | | |
|---|---|---|---|---|---|---|
| | | **IU EPO** | **IU EPO** | **IU EPO** | **IU EPO** | **IU EPO** |
| **cm² Wundfläche** | Menge Gel (g) | (100 IU /g Gel) | (150 IU/g Gel) | (200 IU/g Gel) | (300 IU/g Gel) | (500 IU/g Gel) |
| 1 | 1 | 100 | 150 | 200 | 300 | 500 |
| 10 | 10 | 1.000 | 1.500 | 2.000 | 3.000 | 5.000 |
| 100 | 100 | 10.000 | 15.000 | 20.000 | 30.000 | 50.000 |
| 200 | 200 | 20.000 | 30.000 | 40.000 | 60.000 | 100.000 |
| 300 | 300 | 30.000 | 45.000 | 60.000 | 90.000 | 150.000 |
| | | | | | | |

| (C) 2g Gel / cm² Wundfläche | | | | | | |
|---|---|---|---|---|---|---|
| | | **IU EPO** | **IU EPO** | **IU EPO** | **IU EPO** | **IU EPO** |
| **cm² Wundfläche** | Menge Gel (g) | (100 IU /g Gel) | (150 IU/g Gel) | (200 IU/g Gel) | (300 IU/g Gel) | (500 IU/g Gel) |
| 1 | 2 | 200 | 300 | 400 | **600** | 1.000 |
| 10 | 20 | 2.000 | 3.000 | 4.000 | 6.000 | 10.000 |
| 100 | 200 | 20.000 | 30.000 | 40.000 | 60.000 | 100.000 |
| 200 | 400 | 40.000 | 60.000 | 80.000 | 120.000 | 200.000 |
| 300 | 600 | 60.000 | 90.000 | 120.000 | 180.000 | 300.000 |
| | | | | | | |

| (D) 3g Gel / cm² Wundfläche | | | | | | |
|---|---|---|---|---|---|---|
| | | **IU EPO** | **IU EPO** | **IU EPO** | **IU EPO** | **IU EPO** |
| **cm² Wundnäche** | Menge Gel (g) | (100 IU /g Gel) | (150 IU/g Gel) | (200 IU/g Gel) | (300 IU/g Gel) | (500 IU/g Gel) |
| **1** | **3** | **300** | **450** | **600** | **900** | **1.500** |
| 10 | 30 | 3.000 | 4.500 | 6.000 | 9.000 | 15.000 |
| 100 | 300 | 30.000 | 45.000 | 60.000 | 90.000 | 150.000 |
| 200 | 600 | 60.000 | 90.000 | 120.000 | 180.000 | 300.000 |
| 300 | 900 | 90.000 | 135.000 | 180.000 | 270.000 | 450.000 |

Erfindungsgemäß umfassen die neuen Formulierungen mindestens ein gelbildendes, viskoses hydrophiles Polymer vorzugsweise ein Cellulose-Derivat, vorzugsweise ein Polysaccharid, oder Alginate oder deren Derivate, Chitin oder dessen Derivate oder dessen Salze oder Stärke. Hierbei ist der Ursprung der gelbildenden Polysaccharide unbeachtlich, das heißt, dass diese gelbildenden Polysaccharide pflanzlichen oder tierischen Ursprungs oder auf synthetischem Weg hergestellt sein können. Es ist auch möglich Polysaccharide zu verwenden, die pflanzlichen oder tierischen Ursprungs sind und zusätzlich durch chemische Synthese modifiziert wurden.

Vorzugsweise werden Cellulose-Derivate für die erfindungsgemäßen Gele bzw. Formulierungen eingesetzt. Zu der Gruppe der Cellulose-Derivate zählen im Zusammenhang mit der vorliegenden Erfindung insbesondere Celluloseether und Celluloseester sowie deren Salze. Als Celluloseether kommen hierbei insbesondere Hydroxyalkylcellulosen, wie zum Beispiel Hydroxymethylcellulose, Hydroxyethylcellulose, Hydroxypropylcellulose oder Hydroxybutylcellulose, insbesondere aber Hydroxymethylcellulose oder Hydroxyethylcellulose zum Einsatz. Als Celluloseester kommen hierbei insbesondere Carboxyalkylcellulose, insbesondere Carboxymethylcellulose, Carboxyethylcellulose, Carboxypropylcellulose oder Carboxybutylcellulose, vorzugsweise jedoch Carboxymethylcellulose oder Carboxyethylcellulose zum Einsatz, wobei Carboxymethylcellulose am bevorzugtesten ist.

Gemäß einer weiteren Ausfuhrungsform kann die erfindungsgemäße Formulierung auch mindestens zwei verschiedene gelbildende der oben genannten Polysaccharide, insbesondere der oben genannten Cellulose-Derivate umfassen. Dabei hat es sich als besonders vorteilhaft erwiesen, wenn mindestens eine Verbindung aus der Klasse der Celluloseether und mindestens eine Verbindung aus der Klasse der Celluloseester eingesetzt werden. Besonders bevorzugt hierbei ist eine Formulierung / Hydrogel, das als gelbildende Polysaccharide Hydroxyalkylcellulose und Carboxyalkylcellulose enthält, insbesondere Hydroxethycellulose und Carboxymethylcellulose.

Weiterhin kann ein erfindungsgemäßes Gel als gelbildendes Polysaccharid insbesondere mindestens ein wasserlösliches Cellulose-Derivat umfassen. Diese zeichnen sich dadurch aus, dass sie innerhalb des Gels keine gequollenen Partikel bilden wodurch wiederum ein sehr homogenes Hydrogel resultiert. Zudem zeigt ein wasserlösliches Polysaccharid umfassendes Gel bei der Auftragung auf einer Wunde eine besonders gute Streichfähigkeit auf, bildet zudem eine besonders glatte Oberfläche aus und ist besonders gut modellierbar. Besonders geeignet sind hierbei wasserlösliche Cellulose-Derivate, die nicht quervemetzt sind.

Die erfindungsgemäßen Formulierungen können weiterhin einen Strukturbildner oder eine die Viskosität modulierende Verbindung enthalten. Hierzu sind besonders Polyacrylsäure und deren Salze und insbesondere quervernetzte Polyacrylate geeignet. Beispiele solcher geeigneten Acrylate sind: (Poly)Methacrylat, (Poly)Methylmethacrylat, Polyacrylamid, (Poly)ethoxyethylmethacrylat. Diese Polyacrylsäure-Derivate weisen zudem den Vorteil auf, dass sie einen erheblichen Anteil ihres Eigengewichtes an Wasser aufnehmen können. Durch die Kombination dieser Acrylsäure-Derivate mit mindestens einem gelbildenden Polysaccharid lässt sich somit gezielt ein Gel herstellen, dessen Wasseraufnahme- und Wasserabgabekapazität gesteuert werden kann. Das Verhältnis zwischen Cellulose-Derivat und Acrylsäure-Derivat in der Formulierung kann zwischen 20:1 und 1:1, vorzugsweise aber zwischen 10:1 und 2:1 liegen.

Ferner kann die erfindungsgemäße Formulierung auf ein viskoses insgesamt hydrophil wirkendes Polymer aufgebaut sein, welches eine hydrophobe Träger- oder Stützmatrix, beispielsweise aus Polyurethan oder Polyacrylat aufweist, in welche besagte den Wirkstoff enthaltende Polysaccharide, wie beispielsweise Carboxyalkylcellulosen, eingelagert sind. Derartige Hydrogele sind besonders geeignet für die Wundheilung, weil sie die Wunde feucht halten, ein optimales Milieu für autolytisches Debridement schaffen, und Wundsekrete in den Sekundärverband einlagern. Beispiel hierfür ist Varihesive® Hydrogel.

Generell sind auch andere hydrophobe Polymergerüste oder Stützstrukturen möglich, in dem hydrokolloidale Partikel eines Polysaccharides, wie oben aufgeführt, eingelagert sind.

Prinzipiell sind alle auf Polymerbasis, insbesondere auf Polysaccharidbasis aufgebauten Gel- und Salbengrundlagen, welche sich für die Wundheilung als besonders geeignet erwiesen haben und im Handel erhältlich sind, für die erfindungsgemäße Formulierung geeignet, solange die gleichmäßige langsame Freisetzung von EPO durch Einhaltung von Viskositätswerten wie oben und unten beschrieben, sichergestellt ist.

Die erfindungsgemäßen Formulierungen können weiterhin zusätzlich Elektrolyte enthalten. Geeignete Elektrolyte in Zusammenhang mit der vorliegenden Erfindung sind Verbindungen, die insbesondere beim Auflösen in Wasser, zur Dissoziation in Ionen befähigt sind und die aus mono-, di- und/ oder trivalenten Ionen aufgebaut sind. Diese Elektrolyte können beispielsweise als anorganische oder organische Salze vorliegen und unterscheiden sich von eventuell in der Formulierung vorliegenden Polymeren mit ionischem Charakter. Besonders geeignet sind in diesem Zusammenhang Chloride, Iodide, Sulfate, Hydrogensulfate, Karbonate, Hydrogenkarbonate, Phosphate, Dihydrogenphosphate oder Hydrogenphosphate der Alkali- und Erdalkalimetalle, insbesondere jedoch Natrium-, Kalium- und Kalziumchlorid. Diese Elektrolytgemische simulieren in besonders guter Weise das Elektrolyt-Gemisch in einem von einer Wunde abgegebenen Wundserum. Damit stellen diese Elektrolyte enthaltenden erfindungsgemäßen Formulierungen der Wunde eine besonders wundheilungsfördernde Sphäre zur Verfügung.

In einer weiteren alternativen Ausgestaltung der Erfindung weist die erfindungsgemäße Formulierung zusätzlich ein Polyol auf. Dieses Polyol ist hervorragend als Feuchtigkeitsspender geeignet und stellt somit für die der Wunde umgebenden Haut eine pflegende Komponente dar. Insbesondere sind hierzu Polyole geeignet, die beispielsweise ausgewählt werden können aus der Gruppe bestehend aus: Glycerin (Glycerol), Glykol, Propylenglycol, Polyethylenglykol, Polypropylenglycol, Polyethylenpropylenglykol oder deren Mischungen. Insbesondere können als Polyol in dem vorliegenden Hydrogel Glycerin oder Polyethylenglykol sowie Mischungen hiervon in Mengen zwischen 0.5% bis 10% (w/w) bezogen auf das Gesamtgel eingesetzt werden.

Die erfindungsgemäßen Formulierungen oder Gele, insbesondere Hydrogele, müssen eine Viskosität aufweisen, die dazu geeignet ist, EPO in optimaler Weise zu schützen, aber gleichzeitig gewährleist, dass der Wirkstoff in ausreichender Menge und ausreichend schnell, ohne dass er zuvor metabolisiert wurde, an die Wunde abgegeben werden kann.

Überraschenderweise wurde festgestellt, dass dies in optimaler Weise nur mit Formulierungen sichergestellt ist, die eine verhältnismäßig hohe Viskosität besitzen, was mit einem Gewichtsanteil von quellfähigem Polymer am Gesamtgel, vorzugsweise von Cellulose-Derivate , von mindestens 1.5%, vorzugsweise jedoch 2 - 4%, ganz besonders bevorzugt von 2.5 - 3.5%, insbesondere 3% oder um 3%, erreicht wird. Derartig viskose Gele werden in der Regel für medizinische Zwecke selten eingesetzt, da sie sich meist nicht so gut verarbeiten lassen, wie Gele mit einem Gelbildneranteil von 1 - 2%. Durch Einsatz von beispielsweise Acrylaten kann, wie oben erwähnt, die Verarbeitbarkeit des Gels verbessert werden.

Die erfindungsgemäßen Gele bzw. Formulierungen auf Basis von hydrophilen Polymeren, wie beispielsweise Celluloseethern und / oder Celluloseestern, beispielsweise Carboxyalklcellulosen weisen somit eine dynamische Viskosität von 5.000 bis 100.000 mPa s, vorzugsweise von über 20.000 mPa s, insbesondere von 20.000 bis 70.000 mPa s und ganz besonders von 40.000 bis 60.000 mPa s auf (gemessen Bohlin-Rheometer Typ CSR -10, Kegelspindel 4° / 0 40 mm, oszillometrische Messung, T= 22-25 °C). Ein solches Hydrogel lässt sich ausreichend gut und gleichmäßig über und in einer Wunde verteilen, weist auch bei der Aufnahme von Wundexsudat einen guten Zusammenhalt auf und fließt nicht aus einer zu behandelnden Wunde.

Es ist vorgesehen, dass die erfindungsgemäßen bevorzugten EPO haltigen Formulierungen bzw. Gele folgende Zusammensetzung umfassen: mindestens 70 Gew. % Wasser und 1.5 - 6.0 Gew. % gelbildendes Polysaccharid, vorzugsweise mindestens ein Cellulose-Derivat, insbesondere Carboxymethylcellulose und / oder Hydroxyethylcellulose, und optional 0.1 - 10 Gew. % Acrylsäure-Derivat und / oder 1 - 20% Polyol, vorzugsweise 1 - 5 Gew. % Glycerol, und / oder 0.1 - 5 Gew. % Elektrolyt.

Die erfindungsgemäßen Formulierungen können gegebenenfalls an sich bekannte Hilfsstoffe für die Gelzubereitung inklusive geeignete Konservierungsstoffe enthalten.

Die erfindungsgemäßen Formulierungen bzw. Gele, können auch andere Wirkstoffe aufweisen, die der Wundheilung als solche förderlich sind. Dies ist besonders angezeigt, wenn beispielsweise bakterielle, virale oder Pilzinfektionen aufgetreten sind oder eine Prophylaxe gegenüber derartigen Infektionen vorgesehen ist. Entsprechende für diese Zwecke geeignete Antibiotika, Antimykotika, oder auch Antiphlogisitika sind im Stand der Technik beschrieben. Hierbei sind insbesondere topisch einsetzbare Wirkstoffe geeignet. Beispiele für topisch einsetzbare Antibiotika sind Tetracycline oder Penicilline oder auch Erythromycin, Bacitracin, Tyrothricin, Colistin und Polymxyin B oder Aminoglycoside wie Neomycin, Kanamycin und Paromycin oder Mupirocin.

In einer besonderen Ausführungsform ist der erfindungsgemäßen Formulierung mindestens ein Proteinaseinhibitor beigefügt, welcher die im Wundsekret massiv auftretenden Proteinasen hemmen soll. Als Proteinaseinhibitor ist erfindungsgemäß Aprotinin besonders geeignet, welches die pro-inflammatorische Cytokinausschüttung, die bei einer Verwundung auftritt, zu hemmen vermag. Es hat sich überraschend gezeigt, dass EPO bei topischer Gabe in Anwesenheit von Aprotinin besonders wirksam bei der Wundheilung ist.

Ferner können die erfindungsgemäßen Gele bzw. Formulierungen auch Desinfektionsmittel, wie beispielsweise Povidon-Jod oder dergleichen enthalten.

Neben der erfindungsgemäßen EPO-haltigen Formulierung als solches betrifft die vorliegende Erfindung auch eine Wundauflage, die ein medizinisches Trägermaterial und die Formulierung bzw. das Gel der beschriebenen Art umfasst. Beispielsweise kommen hierbei als Trägermaterial Vliesstoffe oder textile Gewirke oder Gewebe aus natürlichen oder synthetischen Fasermaterialen aber auch Filmabdeckungen, beispielsweise in Form eines Sprühpflasters, zum Einsatz. Hierbei wird das medizinische Trägermaterial mit dem Gel bzw. Hydrogel einseitig oder mehrseitig beschichtet oder imprägniert.

Eine ganz besonders geeignete Wundauflage kann in Form eines speziellen Pflasters zur Verfügung gestellt werden, welches den Wirkstoff EPO in besonders optimierter Weise beinhaltet und ihn an die Wunde freigibt.

Ein solches Pflaster ist in der EP 08 011 985.2 im Detail beschrieben und umfasst eine Trägermatrix, welche den oder die Wirkstoffe enthält, wobei die Trägermatrix, wie folgt, aufgebaut ist:
(i) Bereiche bzw. Strukturen in Form einer oder mehrerer als Behältnis für das Arzneimittel dienenden Kavitäten, und (ii) Bereiche bzw. Strukturen in Form von einer oder mehrerer als kanalähnliche Strukturen dienenden Kavitäten, welche der Aufnahme und Abführung von Wundsekret und / oder Belüftung und / oder der topischen Einbringung von weiteren Wirkstoffen und / oder von Zellen dienen, welche die Heilung der Haut fördern, wobei (a) mindestens ein Bereich gemäß (i) an mindestens einen Bereich gemäß (ii) grenzt, (b) die Bereiche (i) und (ii) auf der von der Wunde abgewandten Seite verschlossen sind, (c) die Bereiche (i) und (ii) auf der der Wunde zugewandten Seite offen oder für die enthaltenen Wirkstoffe, Wundsekret, weitere Wirkstoffe/besagte Zellen zumindest passierbar sind, (d) die als Kavitäten ausgebildeten Bereiche (i) und (ii) in der Ebene des Pflasterfläche angeordnet sind, und (e) die Kavitäten der Bereiche (ii) mindestens eine Öffnung oder einen Anschluss für eine Spritze oder Absaug- / Zuführungsvorrichtung aufweisen, der es ermöglicht, entweder unter Erzeugung eines im Pflaster erzeugten Unterdrucks in den Kavitäten angesammeltes Wundsekret abzuführen und /oder gegebenenfalls besagte weitere Wirkstoffe zu applizieren.

Ein entsprechendes EPO-haltiges Pflaster kann ferner in der Weise ausgestattet sein, dass im Falle nässender oder Wundsekret produzierender Haut oder Wunden (f) die Kavitäten der Bereiche (ii) zusätzlich Drainagemittel aufweisen, die es ermöglichen, in den Kavitäten angesammeltes Wundsekret abzuführen. Dabei können die Kavitäten der Bereiche (ii) Kanäle sein, die untereinander in Verbindung stehen. Die Kavitäten der Bereiche (i) können wannenförmig sein, wobei die offenen Seite der Wannen zur Wunde hin ausgerichtet ist, und die Wannen von rechteckiger, quadratischer, sechseckiger/wabenförmiger oder runder Grundfläche sind. Die besagten Kavitäten der Bereiche (i) sind dabei durch Stege aus dem Material der Trägermatrix voneinander getrennt. Die Stege selbst können ebenfalls kanalähnliche Strukturen aufweisen, welche untereinander zumindest teilweise in Verbindung stehen und der Abführung vom Wundsekret, und / oder der Belüftung und / oder der Zuführung von weiteren Wirkstoffen / besagte Zellen dienen.

Außerdem können die die als Behältnis für das Arzneimittel dienen Kavitäten der Bereiche (i) Substrukturen darstellen, welche in Clustern zusammengefasst sind, die durch Stege aus dem Material der Trägermatrix voneinander getrennt sind. Die Clusterdomänen weisen eine beliebige, rechteckige, quadratische, sechseckige/wabenförmige oder runde Grundflächenform, insbesondere eine quadratische oder rechteckige Grundflächenform auf, wobei ggf. die Form der Clustergrundfläche gleich oder verschieden von der Grundflächenform der Substrukturen der Bereiche (i) sein kann. Dabei kann vorgesehen werden, dass eine Clusterdomäne des Bereiches (i) an mindestens eine Kavität des Bereiches (ii) grenzt und dass ggf. zwei Clusterdomänen der Bereiche (i) durch eine Kavität des Bereiches (ii) von einander getrennt sind.

Die erfindungsgemäßen Formulierungen / Gele, welche gegebenenfalls auf eine im Prinzip bekannte und im Handel erhältliche Wundauflage, oder wie beispielsweise das oben beschriebene Pflaster, aufgebracht sind, können alle 12, 24, 48 oder 72 Stunden, vorzugsweise alle 48 Stunden gewechselt werden, was hauptsächlich durch andere Faktoren und Gegebenheiten, wie beispielsweise Wundsekretbildung, Blutungen oder auftretenden Infektionen, die behandelt werden müssen, notwendig werden kann. Allein aus Gründen der Stabilität und Aktivität des Wirkstoffes EPO innerhalb der erfindungsgemäßen Formulierungsgrundlage ist es aber nicht erforderlich, den Verband oder das Pflaster bis zur vollständigen Wundheilung zu wechseln.

Die erfindungsgemäßen Gele sind nach an sich bekannten Methoden herstellbar. Eines dieser Verfahren ist in den Beispielen näher beschrieben.

Die erfindungsgemäßen EPO - haltigen Gele sind, wie bereits angerührt, besonders bei der Behandlung von Wunden, insbesondere der Haut, der Schleimhaut, im Dentalbereich, bei Schleimhaut-Kieferverletzungen in der Mundhöhle, oder bei Verbrennungen oder Verbrühungen der Haut oder Hauterkrankungen mit begleitenden chronischen Wunden einsetzbar. Wunden der Haut können beispielsweise durch Schnitte, Stiche, Quetschungen, Bisse oder Schussverletzungen hervorgerufen werden, oder können als unvermeidbare Folge von Operationen oder Zahnextraktionen entstehen, Ferner können diverse Krankheiten Wunden der Haut und des Fleisches erzeugen, oder offene Geschwüre ausbilden. Auch bei Transplantationen von Organen oder Amputationen treten größere Wunden auf, die topisch und lokal therapeutisch versorgt werden müssen.

Im Dentalbereich können auch kleinere Wunden bei kariösen Entzündungen und Parondontitis auftreten, die erfolgreich mit den erfindungsgemäßen Formulierungen / Gelen behandelt werden können. Ist die Schmelzoberfläche des Zahns beschädigt, dringen Bakterien weiter in das darunter liegende Dentin vor. In den radiär verlaufenden Dentinkanälen liegen Fortsätze der Pulpa, so dass es im weiteren Verlauf zu einer partiellen oder totalen Infektion und damit Entzündung der Pulpa kommt. Erfolgt keine Behandlung, ist die weitere Folge ein Absterben des Pulpagewebes (Nekrose) und ein bakterieller Zerfall (Gangrän). Werden die gangränösen Massen nicht entfernt, sind Entzündungen außerhalb der Wurzelspitze die Folge. Granulome, Zysten, Fistelbildung oder Abszesse können sich entwickeln. In jeder dieser Stufen kann EPO bzw. das EPO - haltige Gel, vorteilhafterweise nach entsprechender anti-bakterieller Behandlung, erfolgreich eingesetzt werden.

Die erfindungsgemäßen Formulierungen bzw. EPO-haltigen Gele sind insbesondere zur Behandlung von vergleichsweise tiefen Wunden geeignet und lassen sich hervorragend als Wundfüller einsetzen. So lassen sich beispielsweise tiefe dermale Ulzera, die sehr häufig stark nässend sind mit den erfindungsgemäßen Gelen behandeln. Hierbei wird bedingt durch die relativ hohe Viskosität des Gels das Heraussickern von Flüssigkeit aus der Wunde verhindert oder wenigstens verringert. Darüber hinaus können mit dem vorliegenden Gel aber auch trockene Wunden wie beispielsweise trockener Ulcus curis behandelt werden. Hierbei zeigt das vorliegende Gel seine Fähigkeit der Wunde Flüssigkeit zuzuführen und die Entfernung von unerwünschten Substanzen, Belägen und Nekrosen durch eine schonende Debridierung zu gewährleisten. Andere Arten von Wunden, für die die erfindungsgemäße Formulierung angewendet werden kann, umfassen, ohne darauf beschränkt zu sein, Dekubitus Stufe I, II, III (Druckgeschwür), Ulcus cruris (Unterschenkelgeschwür, offenes Bein), diabetisches Fußsyndrom, Hautgeschwüre, Blutgeschwüre, Verbrennungen ersten und zweiten Grades, Schürfwunden und chronische Wunden.

Des Weiteren hierin offenbart ist ein Kit-of Parts, welches mindestens zwei separate Packungen umfasst, wobei die erste Packung die Materialien des Hydrogels bzw. Bestandteilen des Hydrogels, wie Polysaccharid und/oder polymere (hydrophobe) Stützmatrix enthält, und die zweite Packung den Wirkstoff EPO als lagerfähiges Lyophilisat enthält. Der Kit-of-Parts kann die Bestandteile der Formulierungsgrundlage auch, falls sinnvoll, in unterschiedlichen Packungen enthalten. Ebenso kann eine Packung mit einer auf die Menge abgestimmten wässrigen Komponente, in welche die festen Komponenten gelöst oder suspendiert werden können, ein weitere Bestandteil des Kit-of Parts sein, dass heißt, der pharmazeutische Kit kann eine weitere Packungseinheit umfassen, welche das zur Quellung / Polymerisation notwendige Solvenz (vorzugsweise Wasser oder wasserhaltige Mittel) aufweist. Dabei kann der Kit das quellfähige Polymer als Pulver oder in bereits vorgequollener Form enthalten, wobei in letzterem Fall die Viskosität des vorgequollenen Polymers so niedrig zu halten ist (vorzugsweise unter 5.000 mPa s, dass die gleichmäßige Vermischung mit dem Wirkstoff EPO aus der zweiten Packungseinheit gewährleistet ist. Nach Zusatz von EPO kann das Trägerpolymer / Gel letztlich mit der gewünschten Viskosität fertig quellen.

Die einzelnen getrennten Packungen können auch physikalisch miteinander in Verbindung stehen in der Weise, dass ihre Inhalte durch einfache Mechanismen, wie Durchstechen, Durchbohren, Durchdrücken, Abreißen oder Ähnliches miteinander gemischt werden können, beispielsweise in Form eines "Mixed-Closure-Systems". Auf diese Art und Weise, kann insbesondere der Wirkstoff EPO, welcher in lyophilisierter Form unter Beibehaltung seiner Aktivität sehr lange lagerfähig ist, erst bei Bereitung eines frischen Gels im vorgequollenen Zustand niedriger Viskosität unmittelbar vor Auftragung auf die Wunde dem noch nicht auspolymerisiertem Gel bzw. der Formulierungsgrundlage beigesetzt werden, so dass keine Aktivitätsverluste auftreten können, wie dies unter Umständen bei von Anfang an komplett fertigen wirkstoffhaltigen Formulierungen der Fall sein kann.

Die folgenden Beispiele sollen die Erfindung näher beschreiben ohne sie aber in irgend einer Weise zu beschränken. Insbesondere ist der Fachmann in der Lage, mit Hilfe seines Allgemeinwissens die Erkenntnisse aus diesen Beispielen gegebenenfalls zu verallgemeinern. Ferner sind die in den Beispielen angegebenen Substanzen inklusive ihrer Parameter, Eigenschaften, physikalischen Größen, Daten, sowie die beschriebenen speziellen Methoden lediglich exemplarisch zu verstehen, welche der Fachmann, sofern nichts anderes gesagt oder vernunftmäßige bzw. technisch-wissenschaftliche Gründe dagegenstehen, auch verallgemeinern und in andere Zusammenhänge zu bringen in der Lage ist, als in den Beispielen angegeben.

### BEISPIELE

### Beispiel 1:

Erythropoietin (EPO) wird in der Form des Fertigarzneimittels verwendet (NeoRecormon 10 000 IU, Pulver und Lösungsmittel zur Herstellung einer Injektionslösung in Patronen, Ch,-B. MH68260 08, PZN 742 914 3, Roche Reg. Ltd., UK-Welwyn Garden City).
Der Wirkstoff wird in eine vorsterilisierbare Zubereitung unter aseptischen Bedingungen überführt unter Verwendung folgende Gelbildner:
o Hydroxyethylcellulose Ph, Eur. 5.1 (Handelsname: Hydroxyethylcellulose 250 HX Pharm, Ch.-B. 06E29-N01, Fagron, D-Barsbüttel)
o Carmellose-Natrium / Carboyxmethylcellulose Ph. Eur. 5.0 (Handelsname Tylopur C600, Ch.-B. 516 762 65, Caelo, D-Hilden)
o Methylcellulose / Hydroxypropylmethylcellulose USP (Handelsname Metolose 90 SH-100, Ch.-B. 206314, Shin-Etsu, D-Mülheim)
o Povidon Ph. Eur. 5.0 (Handelsname Kollidön 25, Ch.-B. 74-0915, BASF, D-Ludwigshafen)
o Varihesive® , Convatec, Varihesive E® ist ein Hydrokolloidverband. Er besteht innen (auf der Wunde aufliegend) aus einer Hydrokolloidschicht basierend auf einem quellbaren Polysaccharid, die in eine adhäsive Polymermatrix eingelagert ist, und außen aus einem filmbeschichteten Polyurethanschaum.

Neben Wasser für Injektionszwecke als Bulk (Ph, Eur 5.0) kommt als Hilfsstoff Glycerol zum Einsatz (85% oder wasserfrei, Ch.-B. 058 006 2, Fisher Scientific, UK-Loughborough). Als Primärpackmittel zur Aufnahme der Formulierungen werden Fiolax-Injektionsflasehen, Glasart I, eingesetzt (Münnerstädter Glaswarenfabrik GmbH, D-Münnerstadt). Diese werden mit Pharma-Fix-Dichtscheiben 20 mm abgedeckt und mit 21-mm-Metallbördelverschlüssen verbördelt (beides VWR, D-Hannover). Es werden für die Freigabeuntersuchungen organotypische Hautmodelle eingesetzt, deren Herstellung, Kultivierung und Verwendung entsprechend folgender Literaturstelle vorgenommen wurden: C. Hoffmann, C. C. Müller-Goymann, "Use of artificial skin constructs in permeation studies of clindamycin phosphate", Pharmazie 60 (2005) 350-353.

### Beispiel 2: Formulierung wirkstofffreier Hydrogele

Der zu untersuchende Gelbildner wird unter keimarmen Bedingungen in einer tarierten Fantaschale eingewogen und mit der vorgesehenen Menge an Glycerol angerieben. Nach der portionsweisen Zugabe von Wasser für Injektionszwecke als Bulk lässt man die Zubereitung mindestens 2 Stunden lang quellen, bevor verdunstetes Wasser ergänzt wird und das Hydrogel in der Fantaschale homogenisiert wird. In Anteilen zu je 3,3 g wird das Hydrogel in eine heißluftsterilisierte Injektionsflasche überführt, was je nach Konsistenz der Zubereitung durch Aufziehen mit einer Spritze oder durch Umfällen in eine Unguator-Kruke und anschließendes Dosieren mithilfe eines aufgesetzten Unguator-Applikators (beides Gako Konietzko GmbH, D-Bamberg) erfolgen kann. Das verschlossene Injektionsvial wirde dann unter Standardbedingungen der Ph. Eur. mittels gesättigten, gespannten Wasserdampfs sterilisiert.

### Beispiel 3: Evaluierung der rheologischen Eigenschaften der wirkstofffreien Hydrogele

Um den thermischen Abbau der Gelbildner während des Autoklavierens einschätzen zu können, werden sterilisierte Hydrogele mit nicht sterilisierten Proben visuell begutachtet und am Kegel-Plätte-Viskosimeter (Rheometer GVO, Bohlin Instruements, GB-Cirencester) bei einer Temperatur von 20,0 °C und einem Kegelöffnungswinkel von 1° vermessen. Die Festlegung der finalen Gelbildnergehaltes erfolgt entsprechend den therapeutischen Ansprüchen an die Formulierung.

### Beispiel 4: Herstellung einer EPO haltigen gelartigen Formulierung

Aus der rekonstituierten EPO 10.000 IU-Lösung werden unter einer Sterilwerkbank jeweils 500 IU EPO in das abgekühlte und vorsterilisierte Hydrogel eingespritzt; Dazu wird ein Reco-Pen (Roche Diagnostics GmbH, D-Mannheim) genutzt, der die benötigte Arzneistofflösung über eine aufgesetzte Clickfine Universalnadel 12 mm (Ypsomed, D-Sulzbach) abgibt. Unter Berücksichtigung des überführten EPO-Volumens von 50 µl für 500 IU EPO ergibt sich in der Zubereitung ein Wirkstoffgehalt von 150 IU / g. Um unter Ausschluss eines Risikos der Protein-Denaturierung eine homogene Verteilung gewährleisten zu können, ließ man die EPO-Lösung in das Hydrogel über 24 Stunden eindiffundieren. Zur verbesserten Durchmischung wurde das Injektionsvial in dieser Zeit zwei Mal um 180° gedreht. Die Zubereitungen wurden bei +2 bis +8 °C vor Licht geschützt gelagert. Im einzelnen wird folgende Herstellungsanweisung für ein Hydrogel mit einem Erythropoietin-Gehalt von 150 IU / g Gel beispielhaftverwendet:
- Herstellung des wirkstofffreien Gels unter keimarmen Bedingungen.
- Carboxymethylcellulose 250 HX mit Glycerol (2.4%) in einer Fantaschale anreiben
- Portionsweise Wasser einarbeiten
- Mindestens 2 Stunden quellen lassen
- Verdunstetes Wasser ergänzen, homogenisieren. Alternativ kann auch ein elektronisches Rührsystem (z.B. Cito-Unguator 2000) zur Herstellung des wifkstofffreien Gels eingesetzt werden
- Gele in Portionen zu jeweils 3,3 g in ein zuvor gereinigtes und heißluftsterilisiertes Injektionsvial der Glasart 1 überführen und verbördeln, z.B, mit Spritze oder Unguator-Applikator ®
- Verbördeltes Gel unter Standardbedingungen autoklavieren
- Injektionsvials vor der weiteren Verarbeitung auf eine Temperatur von +2 - +8 °C bringen.
- Unter einer Sterilbank jeweils 500 U EPO in jedes Injektionsvial überführen, z.B. mit aus NeoRecormon 10 000 E Zweikammerpatronen mit dem RecoPen unter Abgabe von 2 Pen-Einheiten über eine Clickfine Universalnadel
- EPO über mindestens 24 h eindiffundieren lassen, während dieser Zeit zur besseren Durchmischung das Gel im Injektionsvial zwei Mal um 180° drehen.
In analoger Weise werden Gele mit Hydroxethylcellulose, Carboxyethycellulose, Carboxypropylcellulose, Hydroxpropycellulose (zwischen 1.5% und 3.5% (w/w) Celluloseanteil) hergestellt.

### Beispiel 5: Stabilitätsprüfung von EPO haltigen Formulierungen

Zur Prüfung der Stabilität von Erythropoietin in Zubereitungen werden zum Zeitpunkt 0, 1, 2, 3 und 4 Wochen nach Herstellung den jeweiligen Zubereitungen Proben entnommen und der Erythropoietin-Analytik zugeführt. Während des gesamten Versuchszeitraums werden die oben genannten Lagerbedingungen eingehalten. Außerdem wird auch eine Zubereitung mit einem Erythropoietin-Gehalt von 40 IU / ml in 0,9 % wässriger Kochsalzlösung in die Studie eingeschlossen. Die Ergebnisse sind graphisch in Abb. 1 wiedergegeben.

### Beispiel 6: Wirkstoff Freisetzungsversuche durch Hautkonstrukte

Für die Freigabeversuche werden sechs Franz-Zellen verwendet (Volumen: 5,68 - 8,88 cm³, Permeationsfläche: 0,14-0,34 cm²), die im Akzeptor-Kompartiment eine auf 37 °C temperierte isotone Glycerollösung enthalten. Zwischen Donor- und Akzeptorkompartiment wird das organotypische Hautkonstrukt eingebracht, welches nach unten hin durch einen Polycarbonatfilter gegenüber der Akzeptorlösung mechanisch stabilisiert wird und nach oben hin mit der zu prüfenden Zubereitung in Kontakt steht. Unter Ersatz des entnommenen Akzeptorvolumens von 1,0 ml werden nach 0; 0,5; 1; 1,5; 2; 3; 4; 5; 6 und 24 Stunden Proben gezogen und der Erythropoietin-Analytik zugeführt. Für den Zeitraum zwischen dem ersten Kontakt von Hautkonstrukt mit der zu prüfenden Zubereitung und dem ersten Probenzug (0 h) ist eine Kontaktzeit von etwa 5 Minuten anzusetzen, die für den Zusammenbau der Zelle, das Einfüllen des Akzeptors und Wägungen aufgewendet wird. Vier der oben genannten Freisetzungszellen werden genutzt, um diejenige Formulierung zu prüfen, die aufgrund von Formulierungs- und Stabilitätsstudien als aussichtsreichste Formulierung betrachtet werden kann. Um Kenntnis darüber zu erhalten, ob während der Freigabeprüfung aufgrund der über die Lagerbedingungen hinaus erhöhten Temperatur ein Abbau des Arzneistoffs zu befürchten ist, werden zwei weitere Zellen mit einer viskosen Lösung (Erythropoietingehalt: 150 IU / g Hydrogel) beschickt, um aus diesen Lösungen heraus nach Abschluss der Freigabeversuche den Gehalt bestimmen zu können.

### Beispiel 7: EPO-Anlaytik

Da in allen Fällen die nachzuweisenden Arzneistoffmengen zu gering sind, um eine Quantifizierung des nicht denaturierten bzw. nicht abgebauten Erythropoietin-Anteils mittels des optischen Zirkulardichroismus vorzunehmen, werd Erythropoietin mittels eines Sandwich-ELISA unter Beachtung der Herstelleranweisung nachgewiesen (EPO-ELISA, OSTEOmedical GmbH, D-Bünde). Zur Vermessung des entwickelten ELISA-Assays wird ein Multiplattenreader KC4 (Bio-Tek, D-Bad Friedrichshall) mit der Softwareversion 3.4 Rev 21 genutzt. Da die Gehalte der hergestellten Darreichungsformen oberhalb des Quantifizierungsbereichs des Assays liegen, werden die jeweiligen Proben vor der Vermessung verdünnt, um einen Nenngehalt im Bereich von 400 mIU / ml zu erhalten. Proben aus den Freigabeuntersuchungen können ohne zwischengelagerten Verdünnungsschritt vermessen werden.

### Beispiel 8: Hautregeneration bei traumatiserten Ratten mit einem Carboxymethylcellulose-Gel

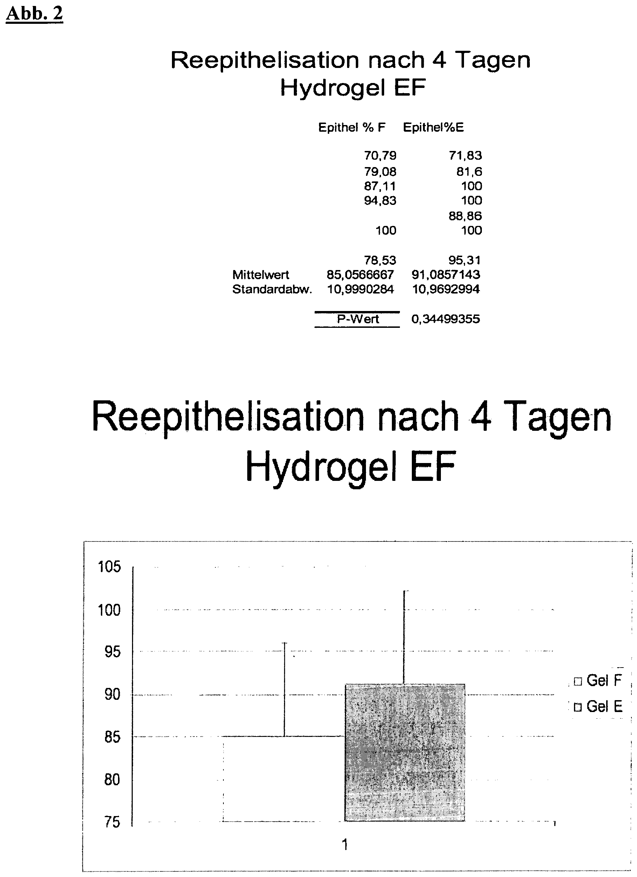

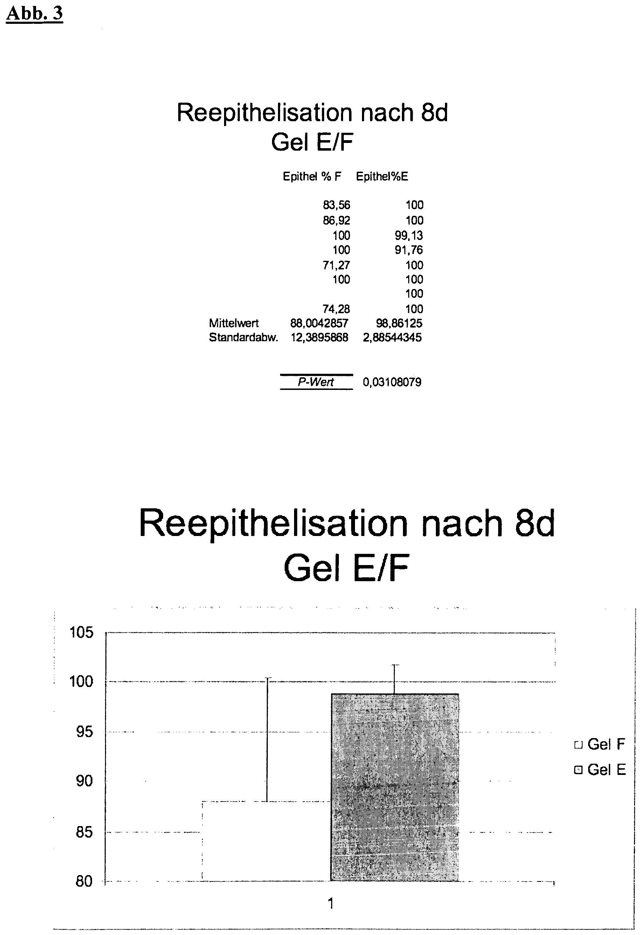

Bei vier weiblichen Ratten mit einem Körpergewicht von 200 - 250 g wird ein tangentiales Exzisionstrauma gesetzt. Die Tiere werden mit einem nach obigen Angaben hergestelltes Gel, welches als Gelbildner Carboxymethylcellulose (3% (w/w), 42.000 mPa s) enthält (ferner 2,4% Glycerol) sowie 150 IU EPO / KGW, behandelt. Die Kontrollen (Placebo) enthalten keinen Wirkstoff. Nach Auftragen des Gels erhatlen die Tiere einen Verband bzw. ein Pflaster. Der Wechsel des Verbandes/Pflasters wird alle 48h vorgenommen. Nach 4 und 8 Tagen werden Gewebeproben aus dem Wundbereich entnommen und histologisch untersucht. Hierbei wird mittels im Stand der Technik bekannter Färbungstechniken auf CD31 - und Nestin - positive, bzw. negative Zellen getestet. Die Ergebnisse sind in den Abb. 2, 3 und 4 dargestellt. Man kann erkennen, dass die Reepithelialiesierung in den EPO-haltigen Gelen ("Gel E", Abb. 2 und 3)) deutlich rascher abgeschlossen ist (durchschnittlich 10 - 50%) als in den wirkstofffreien Formulierungen ("Gel F", Abb. 2 und 3), je nach Dauer der Behandlung. Überdies kann erkannt werden, dass durch Zellen spezifische Einfärbungen die Neubildung des Epithels im wesentlichen keine neuen CD31 positive Zellen generiert (Abb. 4).

In analoger Weise wird die Hautregeneration in einem 1% *Carboxymethylcellulose-Gel untersucht.* Die Ergebnisse sind bei der EPO-haltigen Gel-Formulierung um etwa 5 - 10% schlechter als bei einem vergleichbaren 3% Gel, während keine wesentlichen Unterschiede bei den Kontrollen zu beobachten sind.

In analoger Weise wird die Hautregeneration in einem 1% und einem 3% Gel auf Basis von Hydroxyethylcellulose mit den gleichen EPO-Konzentrationen untersucht. Es werden vergleichbare oder nur geringfügig schlechtere Ergebnisse gegenüber dem Carboxymethylcellulose-Gel erhalten.

### Beispiel 9:

Gemäß des oben genannten Verfahrens wird folgende Gel-Formulierung zur Behandlung einer Verbrennungswunde (ca. 120 cm²) eines erwachsenen Patienten hergestellt:

| **Bestandteil** | **Gew. %** |
|---|---|
| Hydroxyethylcellulose 250 HX Pharm | 3 |
| **Glycerol (wasserfrei)** | 2,4 |
| **Wasser (für Injektionszwecke)** | 94.6 |

| | |
|---|---|
| 100 g eines solchen Gels wurden aufgetragen und enthielten 12.000 IU EPO und weisen eine Viskosität von ca 40.000 mPa x s auf. | |

### Beispiel 10:

Gemäß des oben genannten Verfahrens wurde folgende Gel-Formulierung zur Behandlung einer Schnittwunde (2 cm²) eines erwachsenen Patienten hergestellt:

| **Bestandteil** | **Gew. %** |
|---|---|
| Hydroxyethylcellulose 250 HX Pharm | 4 |
| **Glycerol (wasserfrei)** | 2,5 |
| **Polyacrylat** | 1.5 |
| **Wasser (für Injektionszwecke)** | 92.0 |

| | |
|---|---|
| 2 g eines solchen Gels wurden aufgetragen und enthielten 1.000 IU EPO. Viskosität: ca. 68.000 mPa x s auf. | |

### Beispiel 11:

Gemäß des oben genannten Verfahrens wurde folgende Gel-Formulierung zur Behandlung einer Verbrennungswunde (ca. 300 cm²) eines erwachsenen Patienten hergestellt:

| **Bestandteil** | **Gew. %** |
|---|---|
| Hydroxyethylcellulose 250 HX Pharm | 2.5 |
| **Glycerol (wasserfrei)** | 2,5 |
| **Polyacrylat** | 2.0 |
| **Wasser (für Injektionszwecke)** | 93.0 |

| | |
|---|---|
| 450 g eines solchen Gels enthielten 50.500 IU EPO und wurden für eine Behandlung auf der Brandwunde verteilt. Viskosität: ca. 35.000 mPa x s auf. | |

### Beispiel 12:

Gemäß des oben genannten Verfahrens wurde folgende Gel-Formulierung zur Behandlung einer Verbrennungswunde (80 cm²) eines erwachsenen Patienten hergestellt:

| **Bestandteil** | **Gew. %** |
|---|---|
| **Carboxymethylcellulose** 250 HX Pharm | 3.5 |
| **Glycerol (wasserfrei)** | 3,5 |
| **Wasser (für Injektionszwecke)** | 92.0 |

| | |
|---|---|
| 50 g eines solchen Gels wurden aufgetragen und enthielten 35.000 IU EPO. Viskosität: ca. 55.000 mPa x s auf. | |

### Beispiel 13:

Gemäß des oben genannten Verfahrens wurde folgende Gel-Formulierung zur Behandlung einer offenen Beingeschwürs (150 cm²) eines erwachsenen Patienten hergestellt:

| **Bestandteil** | **Gew. %** |
|---|---|
| **Carboacymethycellulose** 250 HX Pharm | 3.0 |
| **Glycerol (wasserfrei)** | 2,5 |
| **Polymethacrylat** | 1.0 |
| **Wasser (für Injektionszwecke)** | 93.0 |

| | |
|---|---|
| 150 g eines solchen Gels wurden aufgetragen und enthielten 20.100 IU EPO. Viskosität: ca. 49.000 mPa x s auf. | |

### Beispiel 14:

Gemäß des oben genannten Verfahrens wurde folgende Gel-Formulierung zur Behandlung einer größeren Schleimhaut-Kiefer-Wunde (1 cm²) in Folge eine Zahnextraktion bei einem erwachsenen Patienten hergestellt:

| **Bestandteil** | **Gew. %** |
|---|---|
| **Carboxymethycellulose A380 Aquasorb** | 3.0 |
| **Glycerol (wasserfrei)** | 2,5 |
| **Wasser (für Injektionszwecke)** | 94.5 |

| | |
|---|---|
| 1 g eines solchen Gels wurde aufgetragen und enthielt 1.500 IU EPO. | |

### Beispiel 15:

Gemäß des oben genannten Verfahrens wurde folgende Gel-Formulierung zur Behandlung einer größeren Schleimhaut-Kiefer-Wunde (ca. 10 cm²) in Folge eine Zahnextraktion bei einem erwachsenen Patienten hergestellt:

| **Bestandteil** | **Gew. %** |
|---|---|
| **Carboxymethycellulose A380 Aquasorb** | 2.0 |
| **Hydroxyethylcellulose 250 HX Pharm** | 1.0 |
| **Glycerol (wasserfrei)** | 2,0 |
| **Wasser (für Injektionszwecke)** | 95.0 |

| | |
|---|---|
| 30 g eines solchen Gels wurden appliziert und enthielten 20.500 IU EPO. Viskosität: ca. 23.000 mPa x s auf. | |

### Beispiel 16:

### Chronisch-ischämische Wunden

Chronisch-ischämische Wunden zählen allein in der Deutschland zu den häufigsten chirurgischen Krankheitsbildern und bedürfen einer intensiven interdisziplinären Behandlung. In Deutschland leiden mehr als 3 Millionen Menschen an chronischen Wunden, hinter denen sich in aller Regel drei Wundtypen verbergen: Ulcus cruris, diabetischer Fuß und Decubitus. Die Kosten für die Behandlung chronischer Wunden belaufen sich hierzulande gegenwärtig auf circa 5 Milliarden Euro pro Jahr.
Als klinisch besonders problematisch zu behandeln und leider nicht selten gilt die Kombination von Ulcus arteriosum (periphere Verschlußkrankheit) und Ulcus diabeticum.

Ein 69-jähriger diabetischer Patient mit pAVK Grad IV leidet seit mehr als 12 Monate bestehendem chronisch ischämischem Ulcus malleolaris lateralis Grad III (Abb. 5, oberes Bild). Nach dreimaliger Lokalbehandlung mit rekombinanten EPO (jeweils 3.000 IU) in Varihesive E® Hydrogel mit einer entsprechend hohen Viskosität tritt eine deutliche Bildung von Granulationsgewebe ein (mittleres Bild). Eine vollständige Abheilung der Wunde kann nach 15 Tage nach anschließender Spalthauttransplantation (unteres Bild) beobachtet werden.

### Beispiel 17:

### Dermale Wunden

Jeder Mensch erleidet im Laufe seines Lebens mehr oder weniger häufig mechanische oder thermische dermale Verletzungen, die einer Abheilung bedürfen. Entsprechend groß ist der Markt an lokalen Wundtherapeutika in Form von Flüssigkeiten, Salben oder Verbandsmaterialien.

Problematisch in der evidenzbasierten Analyse betroffener Patienten ist die Tatsache, dass jede Hautwunde unterschiedlich konfiguriert ist. Allerdings gibt es in der Chirurgie eine hochstandardisierte Hautwunde, die dadurch entsteht, daß ein Anteil der Haut von definierter Dicke (meist 0,2 bis 0,3 mm) mit einem speziellen Gerät (Dermatom) abgenommen werden muss, um diese danach an anderer Körperstelle zu transplantieren. Solche Spalthautentnahmestellen (0,3 mm) heilen innerhalb von 10 - 14 Tagen meist folgenlos ab.

Ein 25-jähriger Patient wies eine thermische Verletzung auf, deren Behandlung eine Spalthautentnahme notwendig machte (Abb. 6, linkes Bild). Es erfolgte eine Behandlung der Entnahmestellen mit 1 x 3.000 IU EPO in Hydrogel gemäß einer der oben angegebenen Zusammensetzungen (3% Carboxymethylcellulose). Eine Vollständige Abheilung konnte nach bereits 7 Tagen (Abb.6, rechtes Bild) beobachtet werden.
Ähnliche Ergebnisse wurden mit vier weiteren Patienten, welche Verbrennungen erlitten hatten, erzielt

Der Therapieversuch von Spalthauttransplantat-Entnahmestelle mit lokal appliziertem Erythropoetin wurde an den besagten fünf Patienten wie folgt durchgeführt: Die Spalthauttransplantate wurden jeweils mit einer Dicke von 0,3 mm vom Oberschenkel gehoben. Das Erythropoetin wurde intraoperativ mit Varihesive® Hydrogel, welches als Trägersubstanz fungierte, vermischt und direkt auf die Wunde aufgebracht. Anschließend wurde das Wundareal mit einer perforierten Polyurethanfolie und einem Sekundärverband bedeckt. Die Entfernung des Sekundärverbandes erfolgte nur im Falle von weiteren lokalen Erythropoetin Applikationen. Letztere erfolgten steril, an mehreren Stellen, durch die Polyurethanfolie hindurch. Die Entfernung der Polyurethanfolie erfolgte atraumatisch erst sieben Tage nach der Operation zur Beurteilung der Reepithelialisierung.

## Patentansprüche

1. Gelartige oder viskose Formulierung zur topischen Anwendung bei der Behandlung von Wunden, von Verbrennungen oder Verbrühungen der Haut oder von Hauterkrankungen mit begleitenden chronischen Wunden, umfassend Erythropoietin (EPO) und mindestens ein gelbildendes quellfähiges Polysaccharid in einer Konzentration von 2 - 4 Gew. %, ausgewählt aus der Gruppe bestehend aus
-- Hydroxyethylcellulose
-- Hydroxymethylcellulose
-- Carboxyethylcellulose
-- Carboxymethylcellulose,
wobei, die gelartige oder viskose Formulierung im gequollenen Zustand
(i) eine Viskosität von 20.000 - 60.000 mPa x s aufweist,
(ii) EPO in einer Konzentration von 100 - 500 IU / g enthält,
(iii) in einer Menge auf die traumatisierte Haut aufgetragen wird , die 50 - 1.500 IU EPO / cm² Wundfläche entspricht, und
(iv) EPO in der Formulierung stabilisiert und gleichmäßig und langsam freisetzt.

2. Formulierung zur Anwendung nach Anspruch 1, worin das gebildende quellfähige Polysaccharides in einer Konzentration von 2 - 3 Gew. % vorliegt.

3. Formulierung zur Anwendung nach Anspruch 1 oder 2, wobei der Gelbildner Carboxymethylcellulose ist und 150 IU EPO / g gelartige Formulierung vorliegen für den Einsatz von 1g Gel / cm² Wundfläche.

4. Formulierung zur Anwendung nach einem der Ansprüche 1 - 3, wobei sie in oder auf eine feste Trägermatrix eingebracht ist.

5. Formulierung zur Anwendung nach Anspruch 4, wobei die feste Trägermatrix ein dreidimensional strukturiertes Pflaster ist.

6. Formulierung zur Anwendung nach einem der Ansprüche 1 - 5 bei Verbrennungswunden, Verbrühungen, chronisch ischämischen Wunden und im Dentalbereich.

7. Formulierung zur Anwendung nach einem der Ansprüche 1 - 6, wobei die Formulierung erhältlich ist durch Vermischen von EPO in lyophilisierter gelöster oder suspendierter Form mit dem vorgequollenen Polysaccharid mit einer Viskosität unter 5.000 mPa x s.

8. Formulierung zur Anwendung nach einem der Ansprüche 1 - 7, wobei die Formulierung die Wundheilung durch Reepthelialisierung nach 4 bis 8 Tagen zwischen 92% und 99% bewirkt, verglichen mit zwischen 85% und 87% bei derselben Formulierung ohne EPO unter den gleichen Bedingungen.

9. Formulierung zur Anwendung nach einem der Ansprüche 1 - 8, wobei die Formulierung einen Proteinaseinhibitor oder ein Desinfektionsmittel enthält.

## Claims

1. Gelatinous or viscous formulation for topical use in the treatment of wounds, of burns or scalds of the skin or of skin diseases with accompanying chronic wounds, comprising erythropoietin (EPO) and at least one gel-forming swellable polysaccharide in a concentration of 2 - 4% by weight, selected from the group consisting of
-- hydroxyethylcellulose
-- hydroxymethylcellulose
-- carboxyethylcellulose
-- carboxymethylcellulose,
where the gelatinous or viscous formulation in the swollen state
(i) has a viscosity of 20,000 - 60,000 mPa x s,
(ii) comprises EPO in a concentration of 100 - 500 IU / g,
(iii) is applied to the traumatised skin in an amount which corresponds to 50 - 1,500 IU of EPO / cm² of wound area, and
(iv) stabilises EPO in the formulation and liberates it uniformly and slowly.

2. Formulation for use according to Claim 1, in which the gel-forming swellable polysaccharide is present in a concentration of 2 - 3% by weight.

3. Formulation for use according to Claim 1 or 2, where the gel former is carboxymethylcellulose and 150 IU of EPO / g of gelatinous formulation are present for use of 1 g of gel / cm² of wound area.

4. Formulation for use according to one of Claims 1 - 3, where it has been introduced into or onto a solid support matrix.

5. Formulation for use according to Claim 4, where the solid support matrix is a three-dimensionally structured plaster.

6. Formulation for use according to one of Claims 1 - 5 in the case of burn wounds, scalds, chronic ischaemic wounds and in the dental area.

7. Formulation for use according to one of Claims 1 - 6, where the formulation is obtainable by mixing EPO in lyophilised dissolved or suspended form with the pre-swollen polysaccharide having a viscosity below 5,000 mPa x s.

8. Formulation for use according to one of Claims 1 - 7, where the formulation effects between 92% and 99% wound healing after 4 to 8 days by re-epithelialisation, compared with between 85% and 87% in the case of the same formulation without EPO under the same conditions.

9. Formulation for use according to one of Claims 1 - 8, where the formulation comprises a proteinase inhibitor or a disinfectant.

## Revendications

1. Formulation gélatineuse ou visqueuse pour une utilisation topique au niveau du traitement de plaies, de brûlures ou d'échaudures de la peau ou de maladies de la peau accompagnées de plaies chroniques, comprenant de l'érythropoïétine (EPO) et au moins un polysaccharide gonflable formant un gel selon une concentration de 2 - 4% en poids, choisi parmi le groupe constitué par:
-- hydroxyéthylcellulose
-- hydroxyméthylcellulose
-- carboxyéthylcellulose
-- carboxyméthylcellulose,
où la formulation gélatineuse ou visqueuse dans l'état gonflé
(i) présente une viscosité de 20 000 - 60 000 mPa x s,
(ii) comprend de l'EPO selon une concentration de 100 - 500 IU/g,
(iii) est appliquée sur la peau traumatisée selon une quantité qui correspond à 50 - 1 500 IU d'EPO/cm² de surface de plaie, et
(iv) stabilise l'EPO dans la formulation et la libère uniformément et lentement.

2. Formulation pour une utilisation selon la revendication 1, dans laquelle le polysaccharide gonflable formant un gel est présent selon une concentration de 2 - 3% en poids.

3. Formulation pour une utilisation selon la revendication 1 ou 2, dans laquelle le gélifiant est de la carboxyméthylcellulose et 150 IU d'EPO/g de formulation gélatineuse sont présents pour une utilisation de 1 g de gel/cm² de surface de plaie.

4. Formulation pour une utilisation selon l'une des revendications 1 - 3, dans laquelle elle a été introduite à l'intérieur d'une matrice de support solide ou dessus cette même matrice.

5. Formulation pour une utilisation selon la revendication 4, dans laquelle la matrice de support solide est un pansement/plâtre structuré en trois dimensions.

6. Formulation pour une utilisation selon l'une des revendications 1 - 5 dans le cas de plaies par brûlure, d'échaudures, de plaies ischémiques chroniques et dans la zone dentaire.

7. Formulation pour une utilisation selon l'une des revendications 1 - 6, dans laquelle la formulation peut être obtenue en mélangeant de l'EPO sous forme lyophilisée, dissoute ou en suspension avec le polysaccharide pré-gonflé présentant une viscosité inférieure à 5 000 mPa x s.

8. Formulation pour une utilisation selon l'une des revendications 1 - 7, dans laquelle la formulation réalise entre 92% et 99% de la cicatrisation de la plaie après 4 à 8 jours par re-épithélialisation, par comparaison avec entre 85% et 87% dans le cas de la même formulation sans EPO sous les mêmes conditions.

9. Formulation pour une utilisation selon l'une des revendications 1 - 8, dans laquelle la formulation comprend un inhibiteur de protéinase ou un désinfectant.
